(19) Europäisches Patentamt
European Patent Office
Office européen des brevets

(11) EP 1 940 818 B1

(12) **FASCICULE DE BREVET EUROPEEN**

(45) Date de publication et mention de la délivrance du brevet:
**24.08.2011 Bulletin 2011/34**

(21) Numéro de dépôt: **06807506.8**

(22) Date de dépôt: **24.10.2006**

(51) Int Cl.:
*C07D 333/24* (2006.01) *C07H 3/02* (2006.01)
*C07C 323/65* (2006.01) *A61K 31/381* (2006.01)
*A61P 29/00* (2006.01) *A61P 25/00* (2006.01)
*C07C 323/60* (2006.01)

(86) Numéro de dépôt international:
**PCT/EP2006/067711**

(87) Numéro de publication internationale:
**WO 2007/048787 (03.05.2007 Gazette 2007/18)**

(54) **DERIVES D'AMINO-ACIDES CONTENANT UN GROUPEMENT DISULFANYLE COMME INHIBITEURS MIXTES DE LA NEPRILYSINE ET DE L 'AMINOPEPTIDASE N**

EINE DISULFANYLGRUPPE ENTHALTENDE AMINOSÄUREDERIVATE ALS GEMISCHTE INHIBITOREN VON NEPRILYSIN UND AMINOPEPTIDASE N

AMINOACID DERIVATIVES CONTAINING A DISULFANYL GROUP IN THE FORM OF MIXED DISULFANYL AND AMINOPEPTIDASE N INHIBITORS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **25.10.2005 FR 0510862**
**05.05.2006 FR 0604030**

(43) Date de publication de la demande:
**09.07.2008 Bulletin 2008/28**

(73) Titulaire: **Pharmaleads**
**75013 Paris (FR)**

(72) Inventeurs:
- **ROQUES, Bernard**
**F-75014 Paris (FR)**
- **FOURNIE-ZALUSKI, Marie-Claude**
**F-75011 Paris (FR)**

(74) Mandataire: **Ahner, Francis et al**
**Cabinet Regimbeau**
**20, rue de Chazelles**
**75847 Paris Cedex 17 (FR)**

(56) Documents cités:
**FR-A- 2 651 229**

- **FOURNIE-ZALUSKI M-C ET AL: "MIXED INHIBITOR-PRODRUG AS A NEW APPROACH TOWARD SYSTEMICALLY ACTIVE INHIBITORS OF ENKEPHALIN-DEGRADING ENZYMES" JOURNAL OF MEDICINAL CHEMISTRY, AMERICAN CHEMICAL SOCIETY. WASHINGTON, US, vol. 35, no. 13, 1992, pages 2473-2481, XP002019768 ISSN: 0022-2623**
- **NOBLE F ET AL: "INHIBITION OF THE ENKEPHALIN-METABOLIZING ENZYMES BY THE FIRST SYSTEMICALLY ACTIVE MIXED INHIBITOR PRODRUG RB 101-INDUCES POTENT ANALGESIC RESPONSES IN MICE AND RATS" JOURNAL OF PHARMACOLOGY AND EXPERIMENTAL THERAPEUTICS, AMERICAN SOCIETY FOR PHARMACOLOGY AND, US, vol. 261, no. 1, janvier 1992 (1992-01), pages 181-190, XP000386321 ISSN: 0022-3565 cité dans la demande**
- **NOBLE, FLORENCE ET AL: "Pain-suppressive effects on various nociceptive stimuli (thermal, chemical, electrical and inflammatory) of the first orally active enkephalin-metabolizing enzyme inhibitor RB 120" PAIN , 73(3), 383-391 CODEN: PAINDB; ISSN: 0304-3959, 1997, XP002386725 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne de nouveaux inhibiteurs mixtes de la néprilysine et de l'aminopeptidase N.
On sait que les peptides opioïdes naturels ou enképhalines -(Tyr-Gly-Gly-Phe-Met ou Tyr-Gly-Gly-Phe-Leu) - sont essentiellement dégradés par deux métallopeptidases à zinc, la néprilysine (EC 3.4.24.11) qui clive la liaison Gly3-Phe4 (Nature 276 (1978) 523) et l'aminopeptidase N (EC 3.4.11.2) qui coupe la liaison Tyr1-Gly2 de ces peptides (Eur.J.Pharmacol.117 (1985) 233; revue dans Pharmacological review., 1993, 45, 87-146). On connaît des inhibiteurs mixtes de ces deux enzymes, qui, en protégeant complètement les enképhalines endogènes de leur dégradation enzymatique, révèlent les activités pharmacologiques, en particulier analgésiques et antidépressives, des enképhalines. Les inhibiteurs mixtes, décrits dans l'art antérieur, de ces deux activités enzymatiques, sont des composés à fonction hydroxamate (FR 2 518 088 et FR 2 605 004), des composés aminophosphiniques (FR 2 755 135 et FR 2 777 780) et des dérivés d'aminoacides (FR 2 651 229). Les composés décrits dans ces demandes de brevet présentent une excellente activité in vitro et in vivo après administration par voie intracérébroventriculaire ; ceci a été particulièrement démontré dans le cas des hydroxamates (Eur. J. Phamacol., 102, (1984), 525-528 ; Eur.J.Pharmacol., 165, (1989), 199-207 ; Eur.J.Pharmacol.,192, (1991), 253-262), pour lesquels une activité significative a pu également être démontrée après administration intraveineuse (iv) dans un modèle de rat arthritique (Brain Research, 497, (1989), 94-101). Dans le cas des dérivés phosphiniques et des dérivés d'aminoacides, une bonne activité in vivo a été démontrée après administration par voie iv, lorsque les molécules étudiées ont été solubilisées dans un mélange d'huile, d'éthanol et d'eau (J.Med.Chem., 43, (2000), 1398-1408 ; J.Med Chem., 44, (2001), 3523-3530 ; J.Pharm.Exp.Ther., 261, (1992), 181-190). Cependant, même si un des composés appartenant. à la série des dérivés d'aminoacides s'est avéré relativement soluble dans l'eau (Pain, 73, (1997), 383-391), aucune des molécules précédemment décrites ne possède une solubilité en phase aqueuse et une biodisponibilité suffisantes pour être administrée par voie orale et présenter des réponses analgésiques intéressantes à des doses suffisamment faibles chez l'animal pour être transposées chez l'homme. De même aucune des molécules précédemment citées ne permet une administration intraveineuse puisqu'elles exigent dans les essais sur animaux une solubilisation dans des mélanges incompatibles avec l'administration par cette voie chez l'homme.
L'un des objets de l'invention est de fournir de nouveaux composés hydrosolubles capables d'inhiber conjointement les deux activités enzymatiques responsables de la dégradation des enképhalines et de manifester leurs propriétés pharmacologiques après mise en solution dans un soluté aqueux et injection intraveineuse, sous cutanée, percutanée, intrathécale, intraarticulaire et par voie orale ou nasale.
Il est généralement admis que la barrière hématoencéphalique est plus facilement franchie par des molécules hydrophobes et non polaires. Cependant, de façon inattendue, les molécules hydrophiles qui ont été synthétisées présentent des réponses puissantes dans des tests centraux indiquant l'existence d'une bonne capacité à atteindre les structures cérébrales et ce quelques soient les voies d'administration (à l'exception de la voie locale). Un autre objet de l'invention est de fournir des nouveaux composés qui présentent des propriétés des substances morphiniques, en particulier l'analgésie, les effets bénéfiques sur le comportement (diminution de la composante émotionnelle de la douleur et réponses antidépressives) et des effets périphériques (antidiarréique, antitussique, antiinflammatoire) sans en avoir les inconvénients majeurs (tolérance, dépendances physique et psychique, dépression respiratoire, constipation, nausée). De plus les douleurs inflammatoires et neurogéniques, dont la composante périphérique est importante, sont réduites voire éliminées par les composés de l'invention administrés par voie orale et donc sans que ceux-ci ne soient contraints d'atteindre le système nerveux central. Ce résultant, très intéressant mais inattendu, a été formellement démontré par utilisation d'un antagoniste incapable de rentrer dans le cerveau. Ceci réduit totalement tous les effets dus à la stimulation des récepteurs opioïdes cérébraux par les composés de l'invention, sans altérer les effets analgésiques des composés sur ces douleurs, en particulier neurogénique.

L'invention a plus particulièrement pour objet des composés répondant à la formule (I) suivante :

$$H_2N\text{-}CH(R_1)\text{-}CH_2\text{-}S\text{-}S\text{-}CH_2\text{-}CH(R_2)\text{-}CONH\text{-}R_5$$

dans laquelle:

$R_1$ représente :

- une chaîne hydrocarbonée, saturée ou insaturée, linéaire ou ramifiée, comportant de 1 à 6 atomes de carbones, éventuellement substituée par :
  - * un radical OR, SR ou S(O)R, dans chacun de ces radicaux R représente un hydrogène, une chaîne hydrocarbonée linéaire ou ramifiée de 1 à 4 atomes de carbone, un radical phényle ou benzyle,
  - * un radical phényle ou benzyle,

- un radical phényle ou benzyle éventuellement substitué par :

  * 1 à 5 halogènes, notamment le fluor,
  * un radical OR, SR ou S(O)R, dans chacun de ces radicaux R ayant la même signification que précédemment,

- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde ;

$R_2$ représente :

- un radical phényle ou benzyle, éventuellement substitué par :

  * 1 à 5 atomes d'halogènes notamment le fluor,
  * un radical OR ou SR, dans chacun de ces radicaux R ayant la même signification que précédemment,
  * un groupement amino éventuellement mono- ou di-substitué par un groupement aliphatique, cyclique ou linéaire, de 1 à 6 atomes de carbones,
  * un cycle aromatique à 5 ou 6 atomes,

- un hétérocycle aromatique à 5 ou 6 atomes, l'hétéroatome étant un oxygène, un azote ou un soufre,
- un groupe méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme de N-oxyde ou de S-oxyde.

$R_5$ représente :

a) un radical $CH(R_3)-COOR_4$ dans lequel
$R_3$ représente :

- un hydrogène,
- un groupement OH ou OR, R ayant la même signification que précédemment
- une chaîne hydrocarbonée saturée (alkyle), linéaire ou ramifiée, comportant de 1 à 6 atomes de carbone, éventuellement substitué par un radical OR ou SR, dans chacun de ces radicaux R a la même signification que précédemment,
- un radical phényle ou benzyle, éventuellement substitué par :

  * 1 à 5 halogènes notamment le fluor,
  * un radical OR ou SR, R ayant la même définition que précédemment.

et
$OR_4$ représente

- un radical glycolate $OCH_2COOR'$ ou lactate $OCH(CH_3)COOR'$, dans chacun de ces radicaux R' représente

  • une chaîne hydrocarbonée saturée (alkyle) de 1 à 6 atomes de carbone, linéaire ou ramifiée éventuellement substituée par un groupement alkoxy en. C1 à C3, de préférence un groupe alkyle en C1-C4 éventuellement substitué par un groupe méthoxy
  • un groupement cycloalkyle de C5 à C8, de préférence un groupe cycloalkyle en C5-C6,
  • un groupement phényle, benzyle, hétéroaryle, alkylhétéroaryle

- un groupement OCH(R")O(CO)OR' ou OCH(R")O(CO)R', dans chacun de ces radicaux R' a la même signification que précédemment et R" représente

  • un atome d'hydrogène,
  • une chaîne alkyle en C1-C6 linéaire ou ramifiée éventuellement substituée par un groupement alkoxy de C1 à C3, de préférence un groupe alkyle en C1-C4 éventuellement substitué par un groupe méthoxy,
  • un groupe cycloalkyle de C5 à C8, de préférence un groupe cycloalkyle en C5-C6,
  • un groupement phényle, benzyle, hétéroaryle, alkylhétéroaryle,

- un radical triglycéride $OCH(CH_2OCOR')_2$ ou $OCH_2-CH(OCOR')-CH_2OCOR'$, dans chacun de ces radicaux R' ayant la même signification que précédemment
- un radical glycoside tel que D-glucose, β-D-glucopyranose, α -ou β-galactopyranose,
- un radical sulfonate $OCH_2CH_2(SO_2)CH_3$,
- un radical $OCH(CH_2OH)_2$ ;

ainsi que les sels d'addition desdits composés (I) avec des acides minéraux ou organiques pharmaceutiquement acceptables

L'invention a également pour objet les sels d'addition des composés de formule (I), obtenus avec des acides organiques ou minéraux pharmacologiquement acceptables tels que les phosphates, le chlorhydrate, l'acétate, le méthanesulfonate, le borate, le lactate, le fumarate, le succinate, l'hémisuccinate, le citrate, le tartrates l'hémitartrate, le maléate, l'ascorbate, l'hemifumarate, l'hexanoate, l'heptanoate, l'hippurate, l'hydrocinnamate, le phénylglyoxylate et le nicotinate.

Dans le cadre de la présente invention, l'expression « chaîne hydrocarbonée » désigne des alcanes, des alcènes ou des alcynes. En particulier, l'expression « chaîne hydrocarbonée saturée » désigne des radicaux alkyles comportant dé 1 à 6 atomes de carbone (C1-C6) ou de 1 à 4 atomes de carbone (C1-C4), linéaires ou ramifiés. Comme exemple de radiaux alkyles comportant de 1 à 4 atomes de carbones, on peut citer les radicaux méthyl éthyl, propyl, butyl, isopropyl, 1-méthyl-éthyl, 1-méthyl-propyl, 2-méthyl-propyl. Comme exemple de radicaux alkyles comportant de 1 à 6 atomes de carbones on peut en outre citer les radicaux pentyl, hexyl 1-méthyl-butyl, 1-méthyl-pentyl 2-méthyl-bulyl, 2-méthyl-pentyl, 3-méthyl-butyl, 3-méthyl-pentyl, 4-méthyl-pentyl ou 1-éthyl-propyl, 1-éthyl-butyl, 2-éthyl-butyl. L'expression « chaîne hydrocarbonée insaturée» désigne des radicaux alcényle (au moins une double liaison), par exemple vinyle, allyle ou analogue, ou alcynyle (au moins une triple liaison) comportant de 2 à 6 atomes de carbone, ou de 2 à 4 atomes de carbone, linéaires ou ramifiés.

Le terme « halogène » utilisé ici désigne un chlore, un brome, un iode et un fluor. Comme exemple de noyaux hétérocycliques à 5 ou 6 atomes, aromatiques ou saturés, possédant comme hétéroatome un atome d'azote ou de soufre, on peut citer, mais sans limitation, les radicaux suivants : thiényl, pyrrolyl, imidazolyl, pyrazolyl, isothiazolyl, pyridyl; pyrazinyl, pyrimidinyl, pyridazinyl, pyrrolidinyl, pyrrolinyl, imidazolidinyl, pyrazolidinyl, pyrazolinyl, piperidyl, piperazinyl, thiadiazolyle, les atomes d'azote et de soufre étant éventuellement oxydés sous forme de N-oxyde ou de S-oxyde. Comme exemple de noyaux hétérocycliques à 5 ou 6 atomes, aromatiques ou saturés, possédant comme hétéroatome un atome d'oxygène, on peut citer, mais sans limitation, les radicaux suivants : furyl, pyranyl, isoxazolyl, morpholinyl, furazanyl, oxazolyl, oxazolidinyl, oxazolinyl.

Le radical $R_1$ représente avantageusement un radical alkyle ayant de 1 à 4 atomes de carbone, éventuellement substitué par un radical OR, SR ou S(O)R, dans chacun de ces radicaux R la même signification que précédemment. $R_1$ représente encore plus avantageusement un radical alkyle ayant de 1 à 4 atomes de carbone substitué par un radical SR, R ayant la même signification que précédemment, en particulier R représente une chaîne hydrocarbonée saturée linéaire ou ramifiée de 1 à 4 atomes de carbone.

Le radical $R_2$ représente avantageusement :

- un radical benzyle ou phényle,
- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde.

En particulier, le radical $R_2$ représente un radical benzyle ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde, encore plus avantageusement un radical benzyle ou un un radical méthylène substitué par un radical thiophényle (thiényle).

Le radical $R_5$ est un radical qui permet d'augmenter le caractère hydrophile de l'ensemble de la molécule, qui, autrement, est une molécule plutôt hydrophobe.

Selon une première variante de l'invention, le radical $R_5$ représente un radical $CH(R_3)-COOR_4$.

Dans cette première variante, le radical $R_3$ représente avantageusement un atome d'hydrogène ou un radical alkyle ayant de 1 à 6 atomes de carbone, encore plus avantageusement 1 à 4 atomes de carbone, éventuellement substitué par un radical OR ou SR, dans chacun de ces radicaux R ayant la même signification que précédemment. Le radical $R_3$ représente encore plus avantageusement un atome d'hydrogène ou un radical alkyle ayant de 1 à 6 atomes de carbone, encore plus avantageusement 1 à 4 atomes de carbone, substitué par un radical OH ou SH.

Le radical $OR_4$ représente avantageusement :

- un radical glycolate $OCH_2COOR'$, R' ayant la même signification que précédemment (en particulier R' représente

un groupe alkyle en C1-C4 éventuellement substitué par un groupe méthoxy ou un groupe cycloalkyle en C5-C6),

- un radical OCH(R")O(CO)OR' ou OCH(R")O(CO)R', R' et R" ayant la même signification que précédemment (en particulier R' et/ou R" représente un groupe alkyle en C1-C4 éventuellement substitué par un groupe méthoxy ou un groupe cycloalkyle en C5-C6 ou R" représente un atome d'hydrogène),
- un radical triglycéride $OCH(CH_2OCOR')_2$ ou $OCH_2$-CH(OCOR')-$CH_2OCOR'$, dans chacun de ces radicaux R' ayant la même signification que précédemment
- un radical glycoside tel que D-glucose,
- un radical sulfonate $OCH_2CH_2(SO_2)CH_3$,
- un radical $OCH(CH_2OH)_2$

En particulier, le radical $OR_4$ représente un groupement OCH(R")O(CO)OR' ou OCH(R")O(CO)R', le radical R' représentant une chaîne alkyle en C1-C4 (en particulier le radical éthyle) et le radical R" représentant un radical méthyle, CH $(CH_3)_2$, cyclohexyle ou phényle.

L'invention concerne en particulier les composés suivants :

1-(2-(1-(2,3-diacétoxypropoxycarbonyl)-éthylcarbamoyl)-3-thiophèn-3-ylpropyl disulfanylméthyl)-3-méthylsulfanylpropyl-amine,

1-(2-(1-(2-méthanesulfonyléthoxycarbonyl)-éthylcarbatrioyl)-3-thiophèn-3-ylpropyl disulfanylméthyl)-3-méthylsulfanylpropyl-amine,

1-(2-(1-(1-éthoxycarbonyloxyéthoxycarbonyl))-éthylcarbamoyl)-3-thiophèn-3-yl-propyl disulfanylméthyl)-3-méthylsulfanylpropyl- amine,

1-(2-(1-éthoxycarbonylméthyloxycarbonyléthyl carbamoyl) -3-thiophèn-3-yl-propyl disulfanylméthyl)-3-méthylsulfanylpropyl- amine,

1-(2-(1-(1-éthoxycarbonyloxyéthoxycarbonyl)-2-hydroxypropylcarbamoyl)-3-thiophèn-3-ylpropyldisulfanylméthyl)-3-méthylsulfanylpropyl- amine,

1-(2-(1-(2-acétoxy-1-acétoxyméthyléthoxycarbonyl)-éthylcarbamoyl)-3-thiophèn-3-yl propyldisulfanylméthyl)-3-méthylsulfanylpropyl- amine,

1-(2-(1-(2-hydroxy-1-hydroxyméthyléthoxycarbonyl)-éthylcarbamoyl)-3-thiophèn-3-yl propyldisulfanylméthyl)-3-méthylsulfanylpropyl- amine,

1-(2-(1-(3,4,5,6-tétrahydroxytétrahydropyran-2-ylméthoxycarbonyl)-éthylcarbarnoyl)-3-thiophèn-3-yl-propyldisulfanylméthyl)-3-méthylsulfanylpropyl-amine,

1-(2-(1-(1-éthoxycarbonyloxy-éthoxycarbonyl)-2-hydroxypropylcarbamoyl)-3-phényl propyldisulfanylméthyl)-3-méthylsulfanylpropyl- amine,

1-(2-(1-(2-acétoxy-1-acétoxyméthyl-éthoxycarbonyl)-2-hydroxypropylcarbamoyl)-3-phénylpropyldisulfanylméthyl)-3-méthylsulfanylpropyl- amine,

1-(2-((1-éthoxycarbonyloxy-éthoxycarbonylméthyl)-carbamoyl)-3-phényl-propyl disulfanylméthyl)-3-méthylsulfanylpropyl- aminé.

3-(2-amino-4-méthylsulfanyl-butyldisulfanyl)-2-benzyl-N-(5-éthyl-(1,3,4)-thiadiazol-2-yl)-propionamide

1-(2-((1-éthoxycarbonyloxy-2-méthyl-propoxycarbonylméthyl)-carbamoyl)-3-phényl-propyldisulfanylméthyl)-3-méthylsulfanyl-propyl-amine

1-(2-((cyclohexyl-éthoxycarbonyloxy-méthoxycarbonylméthyl)-carbamoyl)-3-phényl-propyldisulfanylméthyl)-3-méthylsulfanyl-propyl-amine

1-(2-((éthoxycarbonyloxy-phényl-méthoxycarbonylméthyl)-carbamoyl)-3-phényl-propyldisulfanylméthyl)-3-méthylsulfanyl-propyl-amine

3-méthylsulfanyl-1-(3-phényl-2-((1-propionyloxy-éthoxycarbonylméthyl)-carbamoyl)-propyldisulfanylméthyl)-propyl-amine

1-(2-((2-méthyl-1-propionyloxy-propoxycarbonylméthyl)-carbamoyl)-3-phényl-propyldisulfanylméthyl)-3-méthylsulfanyl-propyl-amine

1-(2-((cyclohexyl-propionyloxy-méthoxycarbonylméthyl)-carbamoyl)-3-phényl-propyldisulfanylméthyl)-3-méthylsulfanyl-propyl-amine

3-méthylsulfanyl-1-(3-phényl-2-((phényl-propionyloxy-méthoxycarbonylméthyl)-carbamoyl)-propyl disulfanylméthyl)-propyl-amine

Les composés de formule (I) ont potentiellement de 2 à 9 centres d'asymétrie. Les radicaux $R_1$, $R_2$ et $R_3$ seront introduits de façon à obtenir des enchaînements optiquement purs correspondant à une stéréochimie reconnue par les activités enzymatiques. Les radicaux $R_4$ pourront éventuellement contenir un centre d'asymétrie non résolu.
Les composés de formule (I) sont obtenus:

1° par condensation d'un béta-aminothiol protégé sur la fonction amine par un groupement t-butyloxycarbonyle (Boc) (II) avec un acide mercaptoalcanoïque (III) au moyen du chlorure de méthoxycarbonylsulfonyl en solution dans le THF (tétrahydrofurane), conduisant à IV.

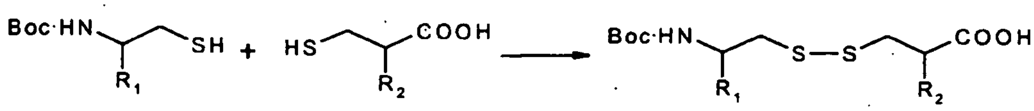

II III IV

Le Boc beta-aminothiol II est préparé à partir du Boc aminoacide commercial correspondant, de configuration absolue S avec rétention de configuration selon un procédé bien connu de l'homme de l'art (J.Med.Chem., 35, (1992) 1259).
L'acide mercaptoalcanoïque III est obtenu à partir du monoester méthylique du malonate correspondant V, qui, selon un procédé bien connu de l'homme de l'art (Ber., 57, (1924),1116) est transformé en acrylate VI.

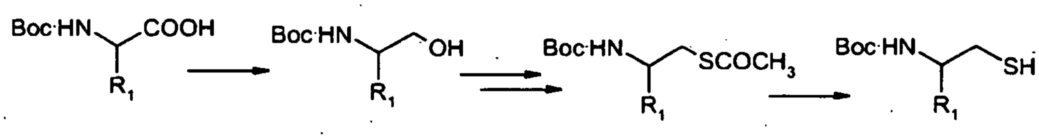

II

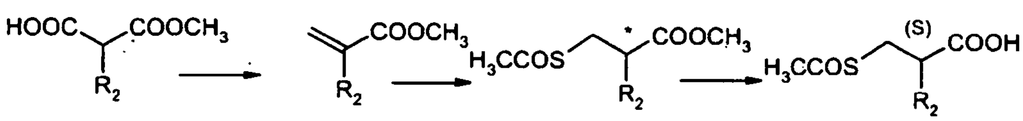

V VI VII VIII

L'addition d'acide thioacétique, à l'acrylate VI, conduit au dérivé VII racémique (Biochemistry, 16, (1977), 5484). Une résolution par l'alpha-chymotrypsine permet d'isoler l'acétylthioacide VIII optiquement pur (Bioorg. Med.Chem.Let., 3, (1993), 2681). L'hydrolyse alcaline du thioester conduit au composé III
2° Les composés de formule (I), dans lesquels le radical $R_5$ représente un radical $CH(R_3)$-$COOR_4$, peuvent être obtenus par les voies de synthèse suivantes.
2.1° Le di-sulfure dissymétrique IV est couplé, dans les conditions classiques du couplage peptidique avec l'aminoester IX, conduisant à l'inhibiteur protégé X.

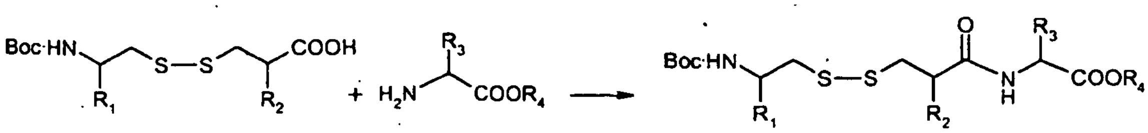

IV IX X

Selon une méthode alternative, les composés X peuvent être obtenus par condensation, au moyen du chlorure de méthoxycarbonyl sulfényl, d'un Boc-β-aminothiol II avec un mercaptoacylaminoester de formule XI.
Le mercaptoacyl aminoester XI est préparé à partir du composé III. Celui ci est oxydé par une solution éthanolique d'iode en di-sulfure XII. Le composé XII est couplé dans les conditions classiques du couplage peptidique avec l'aminoester IX, conduisant à XIII. Le traitement de XIII par un agent réducteur tel que le mélange Zn + HCl 3N, libère le composé XI.

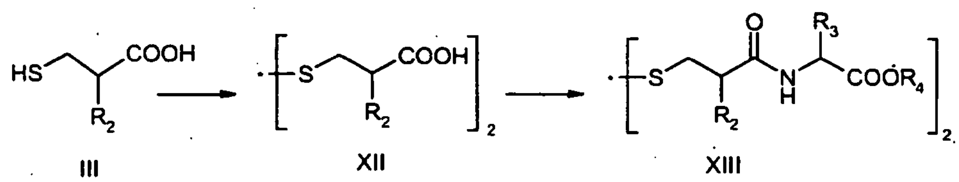

III XII XIII

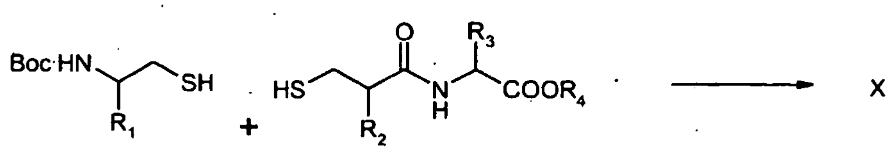

II XI

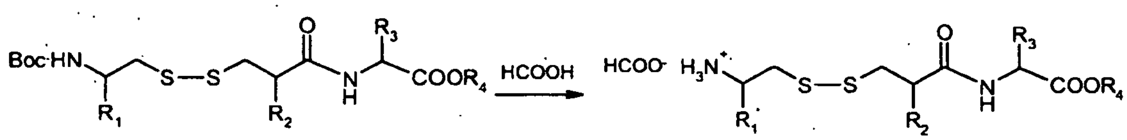


X XIV

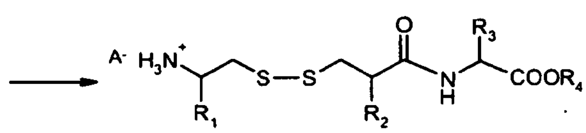

I

La coupure du groupement N-terminal Boc de X est réalisée par action de l'acide formique, libérant XIV. Le changement de contre-ion de XIV est obtenu de façon quantitative par traitement par un équivalent de $NaHCO_3$ 0,1 M, extraction en milieu organique (EtOAc) du composé possédant une fonction aminé libre, puis addition d'un équivalent de l'acide organique ou minéral choisi pour conduire à I.
2.2° L'aminoester IX est obtenu par condensation d'un Boc aminoacide XII avec l'alcool $R_4OH$, puis déprotection par l'acide trifluoroacétique (TFA) et neutralisation par la soude. Si l'alcool $R_4OH$ est un alcool primaire, le couplage avec XII est effectué dans les conditions classiques (hydrochlorure de 1-[3-(diméthylamino)propyl]-3-éthylcarbodiimide (EDCI), 1-hydroxybenzotriazole hydrate (HOBt) ou ester activé). Si l'alcool $R_4OH$ est un alcool secondaire, la condensation se fait par l'intermédiaire d'une réaction de Mitsunobu (Synthesis (1981) 1-28), en utilisant le mélange diéthyl azodicarboxylate / triphényl phosphine (DEAD/$PPhe_3$).

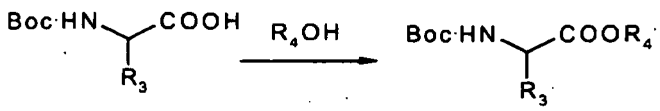

Les alcools $R_4OH$ sont dans la plupart des cas des composés commerciaux. Lorsque $R_4OH$ est un alcool conduisant à un ester "cascade ", celui- ci est synthétisé à partir des méthodes décrites dans la littérature.

L'invention a aussi pour objet les compositions pharmaceutiques contenant à titre de principe actif au moins un des composés de formule générale (I) ou un de ses sels ou hydrates de ses sels en combinaison avec un ou plusieurs support inertes ou autres véhicules pharmaceutiquement acceptables. Ces composés présentent les propriétés des substances morphiniques, en particulier l'analgésie, y compris dans ses composantes périphériques (inflammatoires, neurogéniques), les effets bénéfiques sur le comportement, en particulier en cas de dépression et/ou d'anxiété, sans en avoir les inconvénients majeurs (tolérance, dépendance, dépression respiratoire, constipation). Ainsi à l'encontre des agonistes opioides exogènes interagissant avec les récepteurs delta, les inhibiteurs mixtes selon l'invention ont des effets antidépresseurs sans provoquer de risque de déclenchement de crise épileptiformes ou de convulsions et agissent rapidement (Baamonde A. et al. 1992, Jutkiewicz E.M. et al., 2005). Ces composés sont capables de passer la barrière hémato-encéphalique. La principale application des composés selon l'invention est donc dans le domaine de l'analgésie, des antidépresseurs et des anxiolytiques.

Les compositions pharmaceutiques selon l'invention peuvent être, à titre d'exemple, des compositions administrables par voie orale, nasale (administration par aérosol), sublinguale (administration par diffusion perlinguable), rectale, parentérale intraveineuse et percutanée. A titre d'exemple de compositions administrables par voie orale, on peut citer les comprimés, les gélules, les granules, les microsphères, les poudres et les solutions ou suspensions orales.
Le radical $R_5$ confère une hydrophilie suffisante aux composés selon l'invention, qui sont ainsi solubles dans l'eau et les solvants hydrophiles en présence ou non de divers surfactants. En particulier, ils sont solubles dans les solvants du type alcool/polysorbate/eau, en particulier éthanol/Tween®/eau et mannitol/eau ou à l'aide de cyclodextrines appropriées à l'administration chez l'homme, qui sont fréquemment utilisés pour l'administration par voie intraveineuse. Les compositions selon l'invention peuvent donc être administrées par voie intraveineuse. Elles peuvent également être administrées par voie orale ou nasale, en particulier via un aérosol ou par diffusion perlinguale ou au sein d'une préparation galénique appropriée (microémulsions). De même ces compositions peuvent être utilisées pour des administrations transdermiques. Ces compositions peuvent être utilisées en particulier comme analgésique majeur, comme antalgique puissant dans les douleurs inflammatoires et neurogéniques, et comme anti-dépresseur.
Il est très intéressant de constater que les compositions selon l'invention peuvent être administrées soit sous la forme d'aérosols (microémulsions) par voie orale ou nasale soit par voie intraveineuse. Ces voies d'administration permettent ainsi une administration de la composition selon l'invention par une voie non digestive. Ceci est particulièrement intéressant lorsque la composition comprend des composés complémentaires, qui peuvent présenter des effets non désirés sur le système digestif (en particulier l'intestin), tels que par exemple des dérivés de cannabinoïdes. Ceci permet également d'augmenter la biodisponibilité cérébrale des composés ou associations.
Un autre objet de l'invention est l'utilisation à titre de médicament des composés tels que définis précédemment ou obtenus par un procédé tel que défini précédemment.
Il a en outre été constaté, d'une manière surprenante, que l'association des nouveaux composés selon la présente invention avec des dérivés cannabinoïdes conduit à des effets analgésiques encore plus importants (supérieurs à la somme de chacun des effets pouvant être observé pour chaque composé, ie pour les composés selon l'invention ou pour les dérivés cannabinoïdes).
Jusqu'en 1954. le cannabis a été considéré comme une plante médicinale présentant des propriétés multiples : analgésiques, antispasmodiques, anticonvulsives, anti-inflammatoires, anti-vomitives, broncho-dilatatrices, vasodilatatrices, relaxantes et somnifères. Récemment des propriétés anti-prolifératives et anti-neurodégénératives ont été mises en évidence.
Quelques effets néfastes du cannabis, liés le plus souvent à un surdosage, ont été décrits: crises d'anxiété pour des personnes déprimées et hallucinations lorsque le produit est ingéré par la boisson (tisane) ou la nourriture (gâteaux).
Les effets du cannabis s'expliquent par son action sur les récepteurs cannabinoïdes. Ces récepteurs sont présents dans de nombreuses structures cérébrales et une molécule endogène qui s'y lie naturellement, l'anandamide, a été identifiée.
Deux types de récepteurs ont été caractérisés, les récepteurs CB1 que l'on trouve à la fois dans le système nerveux central et à la périphérie et les récepteurs CB2 qui sont essentiellement périphériques. Les récepteurs CB1 semblent impliqués dans la modulation de la libération neuronale des neurotransmetteurs excitateurs ou inhibiteurs dans le cerveau. Le rôle des récepteurs CB2 est moins clair, mais il semble qu'ils interviennent dans la modulation du système immunitaire.

Les molécules endogènes qui se lient aux récepteurs CB1 et CB2, appelées "endocannabinoïdes", telles que l'anandamide, interagissent avec les récepteurs cannabinoïdes dans le cerveau et à la périphérie en induisant divers effets pharmacologiques.

Le composé psychotrope le plus abondant présent dans le cannabis (cannabis sativa) est le $\Delta^9$ tetrahydrocannabino ($\Delta^9$ THC).

Le $\Delta^9$ THC induit de nombreuses réponses pharmacologiques, telles que l'analgésie, une hypothermie, une activité locomotrice réduite et une perte de la vigilance et de l'attention due à des interactions avec les récepteurs CB1 présents dans le cerveau. Certaines de ces propriétés ont des applications thérapeutiques intéressantes pour le traitement de la douleur et du glaucome, également pour atténuer les nausées et pour stimuler l'appétit de patients traités avec des composés anti-tumoraux et anti-viraux qui présentent de sévères effets secondaires. Le $\Delta^9$ THC, et plus généralement les agonistes du récepteur CB1, sont également capables de réduire les effets douloureux associés aux crises de sclérose en plaque tout en réduisant l'évolution de la maladie. Ceci a conduit néanmoins au développement du SATIVEX qui est une préparation directement issue de la plante (cannabis sativa) et qui contient un mélange à parties égales de $\Delta^9$ THC et de cannabidiol (autre substance présente dans la plante). Cette préparation est actuellement au fin d'exploration clinique. Toutefois les doses administrées par voie orobucale sont élevées et des effets secondaires ont été observés (Current Opinion in Investigational Drugs 2004, 5, 748).

Une autre particularité du système endocannabinoide endogène (anandamide) tient au mode de synthèse et de sécrétion de ce neurotransmetteur particulier. Formé par voie enzymatique à partir des phospholipides des membranes des organelles, l'anandamide est secrété par un transporteur à partir d'un neurone post-synaptique pour interagir avec les récepteurs CB1 situé sur une terminaison présynaptique (neurotransmission rétrograde) (Piomelli et al, TIS, 2000, 21, 218-224).

Toutefois, plusieurs effets comportementaux, tels qu'une perte de la vigilance et de l'attention, une sédation, une ataxie, des troubles de la vision, une tachycardie, des hypothermies et des perturbations comportementales telles que des hallucinations, une anxiété, une attaque de panique et des troubles de la mémoire, produits par une exposition chronique aux cannabinoïdes naturels ou synthétiques, limitent leur utilisation clinique (revue dans E.A. Carlini, the good and the bad effects of (-)transdelta-9-etrahydrocannabinol Δ9THC on humans, Toxicon, 2004, 44, 461-467). De plus, chez l'homme, les effets analgésiques du $\Delta^9$ THC sont obtenus à doses élevées proches des doses donnant les effets indésirables cités plus haut (Campbell F.A. et al., Are cannabinoids an effective and safe treatment option in the management of pain ? A quantitative systemic review, Br. Med. J., 2001, 323, 12-16).

Il a été constaté, d'une manière surprenante, que la co-administration (simultanée ou étalée dans le temps) de faibles doses de dérivés de cannabinoïdes (en particulier, le $\Delta^9$ THC) permet de potentialiser l'effet analgésique et l'effet antidépresseur des dérivés selon l'invention (formule (I)) sans induire, d'une manière significative, les effets néfastes des dits cannabinoïdes, qui par voie iv apparaissent dès 4-5 mg/kg (sédation). Au sens de la présente invention, on entend par l'expression « teneur très faibles en cannabinoïdes » des teneurs en cannabinoïdes inférieures à celles induisants les dits effets secondaires indésirables.

Au sens de la présente invention, on entend par l'expression « cannabinoïdes » le $\Delta^9$ THC, des agonistes du récepteur CB1 synthétiques ou des inhibiteurs de la dégradation de l'anandamide. Les cannabinoïdes introduits dans les compositions selon l'invention sont de préférence le $\Delta^9$THC.

L'invention a également pour objet une composition pharmaceutique comprenant au moins un composé de formule (I) tel que défini précédemment, au moins un dérivé de cannabinoïdes, en particulier le $\Delta^9$ THC, ou un protecteur de son métabolisme (revue Piomelli et al., TIPS, 2000), et un excipient pharmaceutiquement approprié, en particulier un excipient approprié pour une administration par voie orale, nasale, intraveineuse ou transcutanée.

L'invention concerne aussi l'utilisation d'une combinaison d'au moins un composé de formule (I) tel que défini précédemment et d'au moins un dérivé de cannabinoïdes, en particulier le $\Delta^9$ THC, pour la préparation d'un médicament destiné au traitement de la dépression et de la douleur.

Un autre objet de l'invention est une composition pharmaceutique comprenant

i) au moins un composé de formule (I) tel que défini précédemment
ii) au moins un dérivé de cannabinoïdes

en tant que produits de combinaison pour une utilisation simultanée, séparée ou étalée dans le temps.

Dans le cadre de la présente invention, le terme douleur désigne les différents types de douleur, tels que la douleur aigue, la douleur inflammatoire et la douleur neurogénique, y compris la douleur associée à la sclérose en plaque. Les composés selon l'invention, éventuellement en association avec un dérivé de cannabinoïdes, sont également appropriés pour le traitement du glaucome.

L'invention a également pour objet l'association des nouveaux composés selon l'invention avec la morphine ou l'un de ses dérivés. En effet, la morphine est également capable de potentialiser l'effet analgésique induit par les composés selon l'invention. L'invention a donc pour objet une composition pharmaceutique comprenant au moins un composé de

formule (I) tel que défini précédemment, de la morphine ou un de ses dérivés et un excipient pharmaceutiquement approprié, en particulier un excipient approprié pour une administration par voie orale, nasale, intraveineuse ou transcutanée. La composition peut en outre comprendre au moins un dérivé de cannabinoïdes, en particulier le $\Delta^9$ THC, ou un protecteur de son métabolisme.

Cette composition peut être utilisée en tant que médicament, notamment dans le traitement de la dépression et de la douleur. Les différents composés peuvent être utilisés en tant que produits de combinaison pour une utilisation simultanée, séparée ou étalée dans le temps.

C'est la bonne solubilité aqueuse des composés de l'invention selon la formule I qui facilite grandement la constitution de préparation (microemulsions, solution en présence de surfactants ...) appropriée à l'utilisation en thérapeutique par les voies intraveineuse, nasale, pulmonaire (aérosol) ou transcutanée.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière.

L'invention sera encore illustrée par les exemples ci-après. La liste des composés préparés selon l'exemple 12 est donnée dans le tableau 1. Pour tous les composés décrits dans ces exemples.

* $R_1$ représente le radical $-CH_2-CH_2-S-CH_3$.
* $CH_2$-(C:CH.S.CH:CH) représente le radical thiophèn-3-ylméthyl.
* C:CH.CH:CH-CH:CH représente le radical phényle.
* $C_6H_{11}$ représente le radical cyclohexyle

Tableau 1 : radicaux des exemples 12a à 12r.

| Exemple | $R_2$ | $R_3$ | $R_4$ |
|---|---|---|---|
| 12a | $-CH_2$-(C:CH.S.CH:CH) | $-CH_3$ | $-CH_2-C(OCOCH_3)(CH_2OCOCH_3)$ |
| 12b | $-CH_2$-(C:CH.S.CH:CH) | $-CH_3$ | $-CH_2-CH_2-SO_2-CH_3$ |
| 12c | $-CH_2$-(C:CH.S.CH:CH) | $-CH_3$ | $-CH(CH_3)-O-CO-O-C_2H_5$ |
| 12d | $-CH_2$-(C:CH.S.CH:CH) | $-CH_3$ | $-CH_2-CO-O-C_2H_5$ |
| 12e | $-CH_2$-(C:CH.S.CH:CH) | $-CH(OH)CH_3$ | $-CH(CH_3)-O-CO-O-C_2H_5$ |
| 12f | $-CH_2$-(C:CH.S.CH:CH) | $-CH_3$ | $-CH(CH_2OCOCH_3)_2$ |
| 12g | $-CH_2$-(C:CH.S.CH:CH) | $-CH_3$ | $-CH(CH_2OH)_2$ |
| 12h | $-CH_2$-(C:CH.S.CH:CH) | $-CH_3$ | 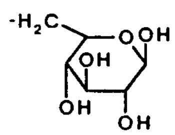 |
| 12i | -C:CH.CH:CH-CH:CH | $-CH(OH)CH_3$ | $-CH(CH_3)-O-CO-O-C_2H_5$ |
| 12j | -C:CH.CH:CH-CH:CH | $-CH(OH)CH_3$ | $-CH(CH_2OCOCH_3)_2$ |
| 12k | -C:CH.CH:CH-CH:CH | -H | $-CH(CH_3)-O-CO-O-C_2H_5$ |
| 12i | -C:CH.CH:CH-CH:CH | -H | $-CH(CH(CH_3))_2)-O-CO-O-C_2H_5$ |
| 12m | -C:CH.CH:CH-CH:CH | -H | $-CH(C_6H_{11})-O-CO-O-C_2H_5$ |
| 12n | -C:CH.CH:CH-CH:CH | -H | -CH(C:CH.CH:CH-CH:CH)-O-CO-O-$C_2H_5$ |
| 12o | -C:CH.CH:CH-CH:CH | -H | $-CH(CH_3)-O-CO-C_2H_5$ |
| 12p | -C:CH.CH:CH-CH:CH | -H | $-CH(CH(CH_3)_2)-O-CO-C_2H_5$ |
| 12q | -C:CH.CH:CH-CH:CH | -H | $-CH(C_6H_{11})-O-CO-C_2H_5$ |
| 12r | -C:CH.CH:CH-CH:CH | -H | -CH(C:CH.CH:CH-CH:CH)-O-CO-$C_2H_5$ |

Légende des figure

Figure 1 : Courbe dose/réponse de l'analgésie induite par la morphine ou le composé 15 injecté par voie i.v. chez la souris (test de la plaque chaude 52°C) ; en abscisses dose en mg/kg et en ordonnées % analgésie.
Le trait noir (trait supérieur) correspond aux résultats obtenus pour la morphine, le trait gris (trait inférieur) correspond aux résultats obtenus pour le composé 15.
**Figure 2 :**
A/ Réponse antinociceptive induite par le composé 15 injecté *per os* 20 min avant le test de la plaque chaude (52°C, latence du saut, seconde) chez la souris OF 1 mâles (*n* = 10) ; en abscisses dose de composé 15 en mg/kg et en ordonnées % analgésie. B/ Cinétique de l'effet du composé 15 après administration per os (n=10-17); en abscisses temps (minutes) et en ordonnées % analgésie.
**Figure 3 :** Réponse antinociceptive induite par l'association du composé 15 et du $\Delta^9$-tetrahydrocannabinol. Plaque chaude (52$\pm$1°C), latence du saut, souris OF1 mâles. Cuff-off: 240 sec. *** $p < 0.001$ versus contrôle, ###, $p < 0.001$ versus composé 15 et $\Delta^9$-THC. ANOVA + Newman-Keuls.

En abscisses doses de composé 15 (0,4 mg/kg), de THC (0,375 mg/kg) et composé 15/THC (0,4 et 0,38 mg/kg) et en ordonnée % analgésie

Exemple 1 : Synthèse de la Boc-méthioninethiol (composé 1)

Ce composé est préparé en suivant le protocole décrit dans J.Med.Chem., 35, 1992, 2473. Solide blanc : mp : 37°C ; Rf (Cyclohexane (CHex), acétate d'éthyle (AcOEt) =1,1) 0,73 ; $\alpha_D^{20°C}$ :-21-,1° (c =1,0 $THCl_3$).

Exemple 2: Synthèse de l'acide (2S)-2-mercaptométhyl-3-phényl propanoique (composé 2)

Etape 1. L'ester méthylique de l'acide 2-acétylthiométhyl-3-phénylpropanoique, obtenu par action de l'acide thioacétique sur l'ester méthylique de l'acrylate correspondant, préparé selon (Ber.,57,1924,1116 ), est traité par l'$\alpha$-chymotrypsine selon le protocole général décrit dans (Bioor.Med.Chem.Let.,3,1993,2681).
Rendement : 71,4% ; excès énantiomérique (ee) : 88%, $\alpha_D^{20°C}$ :-42,7°.

Etape 2. Acide (2S)-mercaptométhyl-3-phénylpropanoique.

Le composé de l'étape 1 est dissous dans du méthanol (MeOH) dégazé à 0°C. On ajoute sous atmosphère inerte 3 équivalents (eq.) de soude (NaOH) 1N. Le mélange est agité 30 min à température ambiante (T.A). Le mélange est acidifié par ajout d'acide chlorhydrique (HCl) 6N (25 ml) et MeOH est évaporé sous pression réduite. La phase aqueuse est extraite par 2x125 ml AcOEt. La phase organique est lavée par une solution de chlorure de sodium saturée (NaCl sat.) puis séchée sur du sulfate de sodium ($Na_2SO_4$) et évaporée à sec. On obtient une huile jaune.
Rendement 100%. HPLC Kromasil C18 $CH_3CN/H_2O$ (0,5 % trifluoroacétate TFA) 60-40 4,96 min.

Exemple 3 : Synthèse de l'acide (2RS) 2-mercaptométhyl-3-thiophèn-3-ylpropanoique (composé 3)

Etape 1 : Un mélange de diméthylmalonate (392 mmol, 45 ml, 1 eq), thiophèn-3-yl aldéhyde (0,357 mmol), pipéridine (1,87 ml ; 0,05 eq) et acide benzoïque (4,58 g ; 0,05 eq) est porté 12h à reflux à l'aide d'un Dean-Stark dans 270 ml de toluène. La phase organique est lavée par 2x140 ml d'HCl 1N, 2$\times$140 ml carbonate de sodium ($NaHCO_3$) 10% et 140 ml de NaCl saturée. La phase organique est séchée sur $Na_2SO_4$ et évaporée à sec. On obtient une huile.
Rendement 100%. HPLC Kromasil C18 $CH_3CN/H_2O$ (0,5% TFA) 60-40: 5,97 min.
Etape 2 Le composé de l'étape 1 (340 mmol) est solubilisé dans MeOH (540 ml). Le mélange est refroidi à 0°C et du borohydrure de sodium ($NaBH_4$) est ajouté peu à peu. Le mélange est agité 15 min à température ambiante. La réaction est stoppée par ajout de 450 ml HCl 1N. Le méthanol est évaporé et le mélange réactionnel est extrait par 2x500 ml de chloroforme ($CHCl_3$). La phase organique est lavée par NaCl sat. puis séchée sur $Na_2SO_4$ et évaporée à sec. On obtient une huile.
poids P=64,1g. Rendement 82,4%.
HPLC Kromasil C18 $CH_3CN/H_2O$ (0,5 % TFA) 60-40: 5,91 min.
Etape 3 Le composé précédent (30 mmol) est dissous dans MeOH (27 ml). Le mélange est refroidi à 0°C et une solution de potasse KOH (1;71g 30,6 mmol) dans MeOH (365 ml) est ajoutée peu à peu. Le mélange est agité 48h à 4°C. Le méthanol est évaporé et le solide obtenu est trituré dans l'éther éthylique $Et_2O$. Le solide obtenu est filtré,

lavé et séché. P=25,2g. Rendement 71,0%.
HPLC Kromasil C18 $CH_3CN/H_2O$ (0,5 % TFA) 60-40: 3,79 min.

Etape 4 Le composé précédent (21,9 mmol) est dissous dans le THF (30 ml). De la diéthylamine $Et_2NH$ (3,0 ml ; 2 eq) et du formaldéhyde à 37% (3,7 ml ; 1,5 eq) sont ajoutés. Le mélange est porté au reflux la nuit. Le THF est évaporé et le mélange est repris dans 90 ml AcOEt. La phase organique est lavée par 3x30 ml HCl 1N, NaCl sat. puis séchée sur $Na_2SO_4$ et évaporée à sec. On obtient une huile incolore.
P=13,1 g. Rendement 72,0%.
HPLC Kromasil C18 $CH_3CN/H_2O$ (0,5 % TFA) 50-50: 14,75 min.

Etape 5 Le composé précédent (72 mmol) est porté à 80°C pendant 5h dans l'acide thioacétique $CH_3COSH$ (10 ml, 144 mmol, 2 eq). L'acide thioacétique est évaporé sous pression réduite. Le mélange est coévaporé plusieurs fois avec du cyclohexane. On obtient une huile orangée. P=18,6 g. Rendement 100%.
HPLC Kromasil C18 $CH_3CN/H_2O$ (0,5 % TFA) 50-50: 17,16 min.

Etape 6. Le composé de l'étape 5 est dissous dans MeOH dégazé à 0°C. On ajoute sous atmosphère inerte 3 eq. de NaOH 1N.Le mélange est agité 30 min à T.A. Le mélange est acidifié par ajout de HCl 6N (25 ml) et MeOH est évaporé sous pression réduite. La phase aqueuse est extraite par 2×125 ml AcOEt La phase organique est lavée par NaCl sat. puis séchée sur $Na_2SO_4$ et évaporée à sec. On obtient une huile jaune.
Rendement 100%. HPLC Kromasil C18 $CH_3CN/H_2O$ (0,5 % TFA) 50-50 6,80 min.

Exemple 4 : Synthèse de l'acide (2S)-2-mercaptométhyl-3-thiophèn-3-ylpropanoique (composé 4)

Etape 1 L'ester méthylique de l'acide 2-acétylthiométhyl-3-thiophèn-3-ylpropanoique, décrit dans l'étape 5 de la synthèse du composé 3, est traité par l'α-chymotrypsine, comme décrit dans la synthèse de 2 (étape 1). Rendement 87,3%. HPLC Kromasil C18 $CH_3CN/H_2O$ (0,5 % TFA) 50-50 7,37 min. ee= 76%

Etape 2 L'acide (2S) acétylthiométhyl-3-thiophén-3-ylpropanoique, obtenu dans l'étape 1, est traité comme décrit dans l'étape 2 du composé 2.
Rendement 97,0%. HPLC Kromasil C18 $CH_3CN/H_2O$ (0,5 % TFA) 50-50 6,80 min.

Exemple 5 : Synthèse de l'acide 2(2S)-Benzyl 3((2S)2t.butyloxycarbonylamino-4-méthylsulfanyl-butyldisulfanyl)- propanoique (composé 5)

Un mélange de 23 ml MeOH et 23 ml THF est refroidi à 0°C sous azote et le chlorosulfonylchloride (1,3 ml, 15,25 mmol, 1,09 eq) est ajouté. Le mélange est agité 15 min à 0°C pour donner le chlorure de méthoxy carbonylsulfényl. Puis, le composé 1 (14,86 mmol, 1,06 eq) dans 16 ml THF/MeOH est ajouté en une seule fois. Le mélange est ramené à température ambiante et est agité 30 min. La solution précédente est ajoutée goutte à goutte à une solution du composé 2 (14,02 mmol, 1 eq) dans 100 ml de $CHCl_3$ dégazé en présence de $Et_3N$ (1 eq). La solution est agitée 1h à température ambiante. Le solvant est évaporé sous pression réduite. Le mélange est repris dans du dichlorométhane $CH_2Cl_2$. La phase organique est lavée : acide citrique 10%, NaCl sat., puis séchée sur $Na_2SO_4$ pour donner un produit brut, qui est chromatographié sur silice avec un mélange Cyclohexane (CHex)/AcOEt 8/2 puis 6/4 comme éluant. P=4,1g Rendement 65,9%. HPLC Kromasil C18 $CH_3CN/H_2O$ (0,5 % TFA) 70-30 : 8,20 min. Exemple 6: Synthèse de l'acide 3-((2S)2-t.butyloxycarbonylamino-4-méthylsulfanyl-butyldisulfanyl)-(2RS)2-thiophen-3-ylméthyl-propanoique (composé 6)
En suivant le protocole décrit pour la synthèse de 5 et en remplaçant le composé 2 par le composé 3, on obtient le composé 6.
Rendement 77,0%. HPLC Kromasil C18 $CH_3CN/H_2O$ (0,5 % TFA) 70-30 : 7,36 min.

Exemple 7 : Synthèse de l'acide 3-((2S)2-t.butyloxycarbonylamino-4-méthylsulfanyl-butyldisulfanyl)-(2S)2-thiophen-3-ylméthyl-propanoique (composé 7)

En suivant le protocole décrit pour la synthèse de 5 et en remplaçant le composé 2 par le composé 4, on obtient le composé 7.
Rendement 77%. HPLC Kromasil C18 $CH_3CN/H_2O$ (0,5%TFA) 70/30 :7,36 min.

Exemple 8 : Synthèse des esters d'alanine

Composé 8a : alanine 2-méthyl sulfonylethyl ester, TFA.
Une solution de 1 eq de BocAlaOH, HOBt (1,2 eq, 879 mg), EDCI (1,2 eq, 1,93 g), $Et_3N$ (triéthylamine), (3 eq, 2,9 ml) dans 10 ml $CH_2Cl_2$ est agitée 12h à température ambiante en présence de 1,2 eq. de 2-méthylsulfonyléthanol en solution dans le $CH_2Cl_2$. Le solvant est évaporé sous pression réduite. Le mélange réactionnel est repris dans AcOEt/ $H_2O$. La phase organique est lavée par acide citrique 10% (2x15 ml), $NaHCO_3$ 10% (2x 15 ml), NaCl saturée, séchée sur $Na_2SO_4$

et évaporée sous pression réduite pour donner un produit brut qui est chromatographié sur silice avec un mélange CHex/AcOEt 8/2 comme éluant pour donner 989 mg de produit.
Rendement: 61,8%. Rf (CHex/AcOEt: 6/4): 0,49.
435 mg (1,488 mmol) de ce produit sont solubilisés à froid dans 2,5 ml de $CH_2Cl_2$ et 1,2 ml de TFA sont ajoutés. Le mélange est agité 2h à température ambiante. Les solvants sont évaporés sous pression réduite. Le mélange est coévaporé par le cyclohexane. Le produit 8a est précipité à froid dans $Et_2O$. Rendement : 100%. Rf ($CH_2Cl_2$/MeOH: 9/1) : 0,25.

Composé 8b : alanine 2,3-diacétoxypropyl ester.

Une solution de 1,026 g (5,428 mmol, 1 eq) de BocAlaOH, HOBt(1,2 eq, 879 mg), EDCI (1,2 eq, 1,93 g), $Et_3N$ (3 eq, 2,9 ml) dans 10 ml $CH_2Cl_2$ est agité 12h à température ambiante en présence de 2,3-diacétoxypropanol (préparé selon Jensen, Topics in Lipid Chemistry, 1972, 3, 1) dans $CH_2Cl_2$. Le solvant est évaporé sous pression réduite. Le mélange réactionnel est repris dans AcOEt/ $H_2O$. La phase organique est lavée par acide citrique 10% (2x15 ml), $NaHCO_3$ 10% (2x15 ml), NaCl saturée, séchée sur $Na_2SO_4$ et évaporée sous pression réduite pour donner 1,62 g d'un produit brut. Le mélange est chromatographié sur silice avec un mélange CHex/AcOEt 8/2 comme éluant pour donner 1,29 g de produit. Rendement : 68,7%. HPLC Kromasil C18 $CH_3CN/H_2O$ (0,5 % TFA) 70-30: 4,25 min.
Ce produit est solubilisé à froid dans 6 ml de $CH_2Cl_2$ et 6 ml de TFA sont ajoutés. Le mélange est agité 2h à température ambiante. Les solvants sont évaporés sous pression réduite. Le mélange est coévaporé par le cyclohexane. Le produit 8b est précipité à froid dans $Et_2O$. P=1,33g. Rendement : 100%. Rf (CHex, EtOAc: 6,4): 0,14.

Composé 8c : alanine 1,3-diacétoxy -2-propyl ester, TFA

1,23 g de 1,3-diacétyl-2-propanol (préparé selon Bentley et McCrae, J.org.Chem, 1971, 35, 2082) (7 mmol, 1,1 eq) est solubilisé dans 50 ml d'$Et_2O$. On ajoute ensuite le diéthyl azodicarboxylate (DEAD) (1,2 eq , 1,1 ml), BocAlanine (5,83 mmol, 1 eq) suivi par la triphénylphosphine ($PPh_3$)(1,2 eq, 1,83 g) et le mélange est agité une nuit à température ambiante. Le solvant est évaporé sous pression réduite. Le mélange est chromatographié sur silice avec un mélange Heptane/AcOEt 8/2 comme éluant pour donner 2,14 g de produit. Rendement : 84,6%. Rf (Hept/AcOEt : 6/4) : 0,42.
2,0 g (4,7 mmol) de ce produit est solubilisé à froid dans 6,5 ml de $CH_2Cl_2$ et 6,5 ml de TFA sont ajoutés. Le mélange est agité 2h à température ambiante. Les solvants sont évaporés sous pression réduite. Le mélange est coévaporé par le cyclohexane. Chromatographie sur silice avec un mélange $CH_2Cl_2$/MeOH/AcOH 9/1/0,5 comme éluant pour donner 1,16 g de composé 8c.
Rendement : 65%. Rf (CH2Cl2/MeOH/AcOH : 9/1/0,5) : 0,22.

Composé 8d : Alanine 1,3 (t.butyl diméthylsilyl)hydroxy -2 propyl ester

La dihydroxyacétone (2 g, 11,10 mmol) est dissoute dans 50 ml de diméthyl formamide (DMF), le chlorure de tert-butyl diméthylsilyl (tBuDMSCl) (4,8 eq, 8,03 g) et l'imidazole (10 eq, 7,56 g) sont ajoutés et le mélange est agité 12h à 20°C. Le mélange est évaporé à sec, repris dans 150 ml AcOEt. La phase organique est lavée par de l'eau $H_2O$ (2x50 ml), HCl 10% (2x50 ml), NaCl saturée, puis séchée sur $Na_2SO_4$ et évaporée sous pression réduite pour donner 20,1 g de produit brut. Le mélange est chromatographié sur silice avec CHex/AcOEt 8/2 comme éluant pour donner 5,96 g de produit. Rendement : 84,5%. Rf ($CH_2Cl_2$/MeOH/AcOH : 9/1/0,5) : 0,24.
Ce produit (8,82 g, 27,73 mmol) est dissous dans THF (74 ml) et $H_2O$ (4,8 ml). Le mélange est refroidi à 5°C et $NaBH_4$ (965 mg, 1 eq) est ajouté peu à peu. Le mélange est agité 30 min à 5°C. L'excès de $NaBH_4$ est détruit par addition d'acide acétique (1 ml). Le THF est évaporé sous pression réduite et le mélange est repris dans $CHCl_3/H_2O$. La phase organique est lavée par $H_2O$ (2x50 ml), $NaHCO_3$ sat. (2x50 ml), NaCl saturée, puis séchée sur $Na_2SO_4$ et évaporée sous pression réduite pour donner 8,24 g de produit. Rendement : 93,0%.
2,93 g de ce composé (9,07 mmol, 1,1 eq) est solubilisé dans 60 ml d'$Et_2O$. Le mélange est agité à température ambiante et DEAD (1,2 eq, 1,56 ml), BocAcïde aminé (8,25 mmol, 1 eq) sont ajoutés suivi par $PPh_3$ (1,2 eq, 2,59 g). Le solvant est évaporé sous pression réduite. Le mélange est chromatographié sur silice avec un mélange CHex/AcOEt 95/5 comme éluant pour donner 4,42 g de produit.
Rendement : 92,9%. Rf (CHex/AcOEt : 9/1) : 0,65.
881 mg (1,79 mmol) de ce produit est solubilisé à froid dans 3 ml de $CH_2Cl_2$ et 1,36 ml de TFA sont ajoutés. Le mélange est agité 2h à température ambiante. Les solvants sont évaporés sous pression réduite. Le mélange est coévaporé par le cyclohexane. Le mélange est précipité à froid dans $Et_2O$ pour donner 920 mg de composé 8d. Rendement : 100%. Rf (CHex/AcOEt : 9/1) : 0,1.

Composé 8e : Alanine carbéthoxyméthyl ester, TFA.

BocAlaOH (5 g, 26,4 mmol) et $Et_3N$ (3,7 ml, 1 eq) sont dissous dans 40 ml d'AcOEt. Le mélange est agité 10 min à température ambiante. L'éthylbromoacétate (6,62 g ; 1,5 eq) est ajouté et le mélange est porté 30 mn à reflux. Le précipité est filtré puis 30 ml $H_2O$ et 50 ml AcOEt sont ajoutés au filtrat. La phase aqueuse est extraite par 3x30 ml AcOEt. La phase organique est lavée par acide citrique 10% (2x30 ml), $NaHCO_3$ 10% (2x30 ml), NaCl saturée, puis séchée sur $Na_2SO_4$ et évaporée sous pression réduite pour donner 7,09 g d'un produit brut. Le mélange est chromatographié sur silice avec un mélange CHex/AcOEt 6/4 comme éluant pour donner 4,68 g de produit.
Rendement : 64,3%. Rf CHex/AcOEt : 6,4) : 0,35.
500 mg (1,81 mmol) de ce produit est solubilisé à froid dans 3 ml de $CH_2Cl_2$ et 1,4 ml de TFA sont ajoutés. Le mélange est agité 2h à température ambiante. Les solvants sont évaporés sous pression réduite. Le mélange est coévaporé par le cyclohexane. Le produit 8c est précipité à froid dans $Et_2O$.
P= 525mg. Rendement : 100%. Rf ($CH_2Cl_2$/MeOH: 95/5) : 0,14.

Composé 8f: alanine éthylcarbonate -1-éthyl ester, TFA.

Boc Ala (76.54 mmol ) et $Et_3N$ (12,27 ml, 1,2 eq) sont dissous dans 70 ml d'AcOEt. Le mélangé est agité 15 min à température ambiante. L'éthyl-1-chloroéthylcarbonate (préparé selon Barcelo et A1. Synthesis, 1986, 627) (14,01 g; 1,2 eq) et iodure de sodium NaI (926 mg, 0,1 eq) sont ajoutés et le mélange est porté 16h à reflux. Le précipité est filtré puis 200 ml $H_2O$ et 200 ml AcOEt sont ajoutés au filtrat. La phase aqueuse est extraite par 3x300 ml AcOEt. La phase organique est lavée par acide citrique 10% (2x150 ml), $NaHCO_3$ 10% (2x150 ml), NaCl saturée, puis séchée sur $Na_2SO_4$ et évaporée sous pression réduite pour donner 24,5g d'un produit brut. Le mélange est chromatographié sur silice avec un mélange CHex/AcOEt 9/1 comme éluant pour donner 18,1 g de produit.
Rendement : 77,51%. Rf ($CH_2Cl_2$/MeOH: 9/1) : 0,33.
9,15g (30 mmol) de ce produit est solubilisé à froid dans 23 ml de $CH_2Cl_2$ et 23 ml de TFA sont ajoutés. Le mélange est agité 2h à température ambiante. Les solvants sont évaporés sous pression réduite. Le mélange est coévaporé par le cyclohexane. Le produit 8f est précipité à froid dans $Et_2O$.
P=9,57g. Rendement: 77,5% (2 étapes). Rf (CHex/AcOEt : 6/4) : 0,1.

Composé 8g : Alanine glucosyl ester, TFA.

Le pentachlorophénol (3 eq, 10 g, 37,54 mmol) est solubilisé à 0°C dans 12 ml AcOEt et le N,N'-Dicyclohexylcarbodiimide (DCC) (2,58g, 12,51 mmol) est ajouté .Le mélange est laissé 12h à -20°C. De l'hexane froid (10 ml) est ajouté au mélange et le solide est filtré, lavé par l'hexane froid. Le solide est recristallisé dans l'hexane pour donner 10,3 g de solide brun. Rendement : 82,0%. Mp : 115-130°C.
Ce complexe est ajouté à 120 ml d'AcOEt. Après dissolution totale, le BocAlanine (1,0 eq, 10,25 mmol) est ajouté et le mélange est agité la nuit à température ambiante. Le solvant est évaporé sous pression réduite puis 100 ml d'$Et_2O$ sont ajoutés. La suspension est refroidie pendant 1h puis le solide est filtré. Celui-ci est mis en suspension dans 100 ml dioxanne, filtré et lavé par 2x20 ml dioxanne. Le filtrat est évaporé à sec. Le résidu est traité à nouveau par dioxanne pour éliminer le dicyclohexylurée (DCU). Il est ensuite mis en suspension dans 100 ml $Et_2O$ mis au congélateur pour la nuit. Le solide est filtré puis séché pour donner 1,29 g de solide brun. Rendement : 27,8%.
A une solution de glucose (3 eq, 1,54 g) dans 57 ml de pyridine redistillée, on ajoute le composé précédent et l'imidazole. Le mélange est agité la nuit à température ambiante. Le solvant est évaporé sous pression réduite pour donner un produit brut. Le mélange est chromatographié sur silice avec un mélange AcOEt/AcOH 20/1 comme éluant pour donner 883 mg de produit. Rendement : 88,3%. Rf(AcOEt/AcOH : 20/1) : 0,13.
883 mg (2,51 mmol) de ce produit est refroidi à 0°C et 61 ml de TFA sont ajoutés. Le mélange est agité 5 min à 0°C puis 30 min à température ambiante. Le TFA est évaporé sous pression réduite. Le mélange est coévaporé par le cyclohexane. Le mélange est précipité à froid dans $Et_2O$ pour donner 746 mg du composé 8g brun.
Rendement : 81,3%. Rf (AcOEt/AcOH : 10/1) : 0,10.

Exemple 9 : Synthèse des esters de la Thréonine

Composé 9a : Thréonine 1,3-diacétyl-2-propyl ester, TFA

Ce composé est obtenu en suivant le protocole décrit pour 8c, et en remplaçant la Boc-alanine par la Boc-thréonine.

Composé 9b : Thréonine éthylcarbonate-1-éthyl ester, TFA.

Ce composé est obtenu en suivant le protocole décrit pour le composé 8f et en remplaçant la Boc-alanine par la Boc-thréonine.
Rendement : 89,7% Rf(Chex/AcOEt :6/4) :0,1.

Exemple 10 : Synthèse des esters de la glycine

Composé 10a Glycine éthyl carbonate-1-ethyl ester, TFA

Ce composé est obtenu en suivant le protocole décrit pour le composé 8f en remplaçant la Boc alanine par la Boc-glycine.
Rendement 92% Rf(Chex/AcOEt 8/2) 0,22

Composé 10b: Glycine éthyl carbonate-1-(-2-méthyl)propyl ester, TFA

Ce composé est obtenu en suivant le protocole décrit pour le composé 10a en remplaçant l'éthyl-1-chloroéthylcarbonate par l'éthyl-1-chloro-2-méthyl-propyl carbonate.
Rendement 88% Rf(Chex/AcOEt 8/2) 0,12

Composé 10c : Glycine éthyl carbonate méthylcyclohexyl ester, TFA

Ce composé est obtenu en suivant le protocole décrit pour le composé 10a en remplaçant l'éthyl-1-chloroéthyl carbonate par l'éthyl-chlorométhyl cyclohexyl carbonate.
Rendement 78 % ; Rf(Chex/AcOEt 7/3) 0,31

Composé 10d : Glycine éthyl carbonate méthylphenyl ester, TFA

Ce composé est obtenu en suivant le protocole décrit pour le composé 10a en remplaçant l'éthyl-1-chloroéthyl carbonate par l'éthyl chlorométhylphényl carbonate.
Rendement 82 % ; Rf(Chex/AcOEt 7/3) 0,46

Composé 10e: Propionic acide 1-(2-amino-acétoxy)-éthyl ester (Gly-OCH($CH_3$)O-COEt)

Ce composé est obtenu par condensation entre la Boc-Gly et le 1-chloroéthyl propionate (1,1eq) en présence de NaI (0,2 eq) et de $Et_3N$ (1,2 eq) dans l'acétate d'ethyle (10 mL/mmol) au reflux pendant une nuit. Après refroidissement, la phase organique est lavée à l'eau, acide citrique 10%, $NaHCO_3$ 10%, $H_2O$, NaCl sat. et séchée sur $Na_2SO_4$.
Après évaporation, on obtient un produit huileux. Rendement 86 %
La déprotection du groupement Boc est effectuée comme décrit dans les exemples précédents. Produit solide blanc Rendement quantitatif.
Rf(Chex/AcOEt 6/4) 0,64

Composé 10f: Propionic acide 1-(2-aminoacétoxy)-2-méthyl propyl ester (Gly OCH(CH($CH_3$)$_2$)o-COEt)

Ce composé est obtenu en suivant le protocole décrit dans l'exemple 10e en remplaçant le 1-chloroéthyl propionate par le 1-chloro-2-méthyl propyl propionate.
Solide blanc Rendement 78% sur les deux étapes. Rf(Chex/AcOEt 6/4) 0,56

Composé 10g : Propionic acide (2-aminoacétoxy)-cyclohexyl-méthyl ester (Gly-OCH(Chex)O-COEt)

Ce composé est obtenu en suivant le protocole décrit dans l'exemple 10e en remplaçant le 1-chloroéthyl propionate par le chlorométhyl(cyclohexyl)propionate.
Solide blanc. Rendement 72% sur les deux étapes. Rf(Chex/AcOEt 6/4) 0,38

Composé 10h: Propionic acide (2-amino-acétoxy)-phényl-méthyl ester (GlyOCH(Ph)O-COEt)

Ce composé est obtenu en suivant le protocole décrit dans l'exemple 10e en remplaçant le 1-chloroéthyl propionate par le chlorométhyl(phényl) propionate.

Solide blanc. Rendement 75% sur les deux étapes. Rf(Chex/AcOEt 6/4) 0,42

Exemple 11 : Synthèse du 2-amino-5 ethyl-(1,3,4)thiadiazole

Un mélange de 25g (0,27 mol) de thiosemicarbazide et de 46,6 mL chlorure de propanoyle (0,54 mol, 2 eq) est agité à 40°C pendant 4 h. L'excès de chlorure de propanoyle est alors évaporé sous vide et le résidu est trituré dans l'éther. Un produit solide est obtenu. Il contient le thiadiazole attendu et une impureté que l'on élimine par précipitation dans l'éthanol. Solide blanc 33,6 g (Rdt 83%) HPLC Kromasil C18 Tr 6,32 min à 30% $CH_3CN$.

Exemple 12 : Synthèse des inhibiteurs mixtes pour lesquels $R_5$=CH($R_3$)$COOR_4$

Le di-sulfure 5;6 ou 7 (0,54 mmol) est solubilisé dans 4 ml de DMF. On ajoute le Benzotriazole-1-yl-oxy-tris-(diméthylamino)-phosphonium hexafluorophosphate (BOP) (1,2 eq ; 1,0 g) et la diisopropyl-éthyl-amine (DIEA) (284 µl) puis l'ester d'amino acide 8, 9 ou 10 (1,3 eq). Le mélange est agité 20 min à température ambiante puis le DMF est évaporé sous pression réduite. Le produit est repris dans AcOEt. La phase organique est lavée par $H_2O$, l'acide citrique 10%, $NaHCO_3$ 10% ; NaCl sat. et séchée sur $Na_2SO_4$.
Le produit brut est purifié par chromatographie sur silice. Le composé obtenu (0,38 mmol) est solubilisé dans 640µl de $CH_2Cl_2$ et 320µl de TFA sont ajoutés. Le mélange est agité 1h à température ambiante. L'excès de solvant est évaporé sous pression réduite. Le mélange est coévaporé avec le cyclohexane. Le mélange est purifié par HPLC semi-préparative ou précipité dans un mélange Hexane/$Et_2O$.

Composé 12a: 1-(2-(1-(2,3-diacétoxypropoxycarbonyl)-éthylcarbamoyl)-3-thiophèn-3-ylpropyldisulfanylméthyl)-3-méthylsulfanylpropyl-ammonium trifluoroacétate (composé 6 ou 7 + composé 8b).

P : 176 mg ; Rendement 66,9%. HPLC Kromasil C18 $CH_3CN/H_2O$ (0,5 % TFA) 40-60 : 9,07 et 10,18 min. ESI : $(M+H)^+$ = 581. Log Kow = 1,31

Composé 12b: 1-(2-(1-(2-méthanesulfonyléthoxycarbonyl)-éthylcarbamoyl)-3-thiophèn-3-ylpropyldisulfanylméthyl)-3-méthylsulfanylpropyl-ammonium trifluoroacétate (composé 6 ou 7 + composé 8a).

P : 200 mg ; Rendement 74,1%. HPLC Kromasil C18 $CH_3CN/H_2O$ (0,5 % TFA) 40-60 : 5,0 et 5,35 min. ESI : $(M+H)^+$ = 529. Log Kow = -0,3

Composé 12c : 1-(2-(1-(1-éthoxycarbonyloxyéthoxycarbonyl))-éthylcarbamoyl)-3-thiophèn-3-yl-propyldisulfanylméthyl)-3-méthylsulfanylpropyl-ammonium trifuoroacétate(composé 6 ou 7 + composé 8f).

P : 232 mg ;Rendement 71,3%. HPLC Kromasil C18 $CH_3CN/H_2O$ (0,5 % TFA) 50-50 : 3,84 et 4,03 min. ESI : $(M+H)^+$ = 509. Log Kow = 1,63

Composé 12d: 1-(2-(1-éthoxycarbonylméthyloxycarbonyléthyl carbamoyl) -3-thiophèn-3-yl-propyldisulfanylméthyl)-3-méthylsulfanylpropyl-ammonium trifluoroacétate (composé 6 ou 7 + composé 8e).

P : 261 mg; Rendement 83,9%. HPLC Kromasil C18 $CH_3CN/H_2O$ (0,5 % TFA) 50-50 : 4,90 et 5,18 min. ESI : $(M+H)^+$ = 539. Log Kow = 1,35

Composé 12e: 1-(2-(1-(1-éthoxycarbonyloxyéthoxycarbonyl)-2-hydroxypropylcarbamoyl)-3-thiophèn-3-ylpropyldisulfanylméthyl)-3-méthylsulfanylpropyl-ammonium trifluoroacétate (composé 6 ou 7 + composé 9b)

P : 285 mg ; Rendement 47,8%. HPLC Kromasil C18 $CH_3CN/H_2O$ (0,5 % TFA) 40-60 : 10,55 et 11,09 min. ESI : $(M+H)^+$ = 594. Log Kow = 0,76

Composé 12f : 1-(2-(1-(2-acétoxy-1-acétoxyméthyléthoxycarbonyl)-éthylcarbamoyl)-3-thiophèn-3-ylpropyldisulfanylméthyl)-3méthylsulfanylpropyl-ammonium trifluoroacétate (composé 6 ou 7 + composé 8c).

P : 171 mg ; Rendement 70,1%. HPLC Kromasil C18 $CH_3CN/H_2O$ (0,5 % TFA) 40-60 : 7,35 et 8,09 min. ESI : $(M+H)^+$ = 581. Log Kow = 1,31

Composé 12g : 1-(2-(1-(2-hydroxy-1-hydroxyméthyléthoxycarbonyl)-éthylcarbamoyl)-3-thiophèn-3-ylpropyldisulfanylméthyl)-3-méthylsulfanylpropyl-ammonium trifluoroacétate (composé 6 ou 7 + composé 8d).

P : 166 mg ; Rendement 67,2%. HPLC Kromasil C18 $CH_3CN/H_2O$ (0,5 % TFA) 40-60 : 2,94 et 3,27 min. ESI : $(M+H)^+$ = 497. Log Kow = -0,29

Composé 12h 1-(2-(1-(3,4,5,6-tétrahydroxytétrahydropyran-2-ylméthoxycarbonyl)-éthylcarbamoyl)-3-thiophèn-3-yl-propyldisulfanylméthyl)-3-méthylsulfanylpropyl-ammonium trifluoroacétate (composé 6 ou 7 + composé 8g).

P : 85 mg ;Rendement 93,2%. HPLC Kromasil C18 $CH_3CN/H_2O$ (0,5 % TFA) 50-50 : 2,34 min. ESI : $(M+H)^+$ = 585. Log Kow = -1,15

Composé 12i : 1-(2-((1-(1-éthoxycarbonylloxy-éthoxycarbonyl)-2-hydroxypropylcarbamoyl)-3-phénylpropyldisulfanylméthyl)-3-méthylsulfanylpropyl-ammonium trifluoroacétate (composé 5 + composé 9b).

P : 1.88g; Rendement 83,8%. HPLC Kromasil C18 $CH_3CN/H_2O$ (0,5 % TFA) 45-55 : 7,0 min. ESI : $(M+H)^+$ = 563. Log Kow = 0,76

Composé 12j :1-(2-(1-(2-acétoxy-1-acétoxyméthyl-éthoxycarbonyl)-2-hydroxypropylcarbamoyl)-3-phénylpropyldisulfanylméthyl)-3-méthylsulfanylpropyl-ammonium trifluoroacétate (composé 5 + composé 9a).

P : 532 mg ; Rendement 53,6%. HPLC Kromasil C18 $CH_3CN/H_2O$ (0,5 % TFA) 40-60 : 6,16 min. ESI : $(M+H)^+$ = 605. Log Kow = 0,44

Composé 12k : 1-(2-(1-éthoxycarbonyloxy-éthoxycarbonylméthyl)-carbamoyl)-3-phényl-propyldisulfanylméthyl)-3-méthylsulfanylpropyl-ammonium trifluoroacétate (composé 5 + composé 10a).

P : 1,76g ; Rendement 89,5%. HPLC Kromasil C18 $CH_3CN/H_2O$ (0,5 % TFA) 50-50 : 5,33 min. ESI : $(M+H)^+$ = 519. Log Kow 1,39

Composé 121:1(2-((1-éthoxycarbonyloxy-2-méthyl-propoxycarbonylméthyl)-carbamoyl)-3-phényl-propyldisulfanylméthyl)-3-méthylsulfanyl-propyl-ammonium, trifuoroacétate (composé 5+ composé 10b)

P:1,2g. Rendement 82,3%-HPLC Kromasil C18 $CH_3CN/H_2O$ (0,5%TFA) 50/50 9,33 min, ESI : $(M+H)^+$ = 547

Composé 12m: 1-2-((cyclohexyl-éthoxycarbonyloxy-méthoxycarbonylméthyl)-carbamoyl)-3-phényl-propyldisulfanylméthyl)-3-méthylsulfanyl-propyl-ammonium, trifluoroacétate (composé 5+composé 10c)

P : 2,1g. Rendement 65,3%. HPLC Kromasil C18 $CH_3CN/H_2O$ (0,5%TFA) 50/50 12,65 min, ESI : $(M+H)^+$=587

Composé 12n : 1-(2-((éthoxycarbonyloxy-phényl-méthoxycarbonylméthyl)-carbamoyl)-3-phényl-propyldisulfanylméthyl)-3-méthylsulfanyl-propyl-ammonium, trifluororacétate (composé 5 + composé 10d)

P : 0,95 g. Rendement 68,1%. HPLC Kromasil C18 $CH_3CN/H_2O$ (0,5%TFA) 50/50 10,86 min. ESI $(M+H)^+$ = 581

Composé12o :3-méthylsulfanyl-1-(3-phényl-2-((1-propionyloxy-éthoxycarbonylméthyl)-carbamoyl)-propyldisulfanylméthyl)-propyl-ammonium, trifluoroacétate (composé 5 + composé 10e)

P : 1,6 g. Rendement 81,2%. HPLC Kromasil C18 $CH_3CN/H_2O$ (0,5%TFA) 50/50 6,82 min. ESI $(M+H)^+$ = 502.

Composé 12p : 1-(2-((2-méthyl-1-propionyloxy-propoxycarbonylméthy))-carbarnoyl)-3-phényl-propyldisulfanylméthyl)-3-méthylsulfanyl-propyl-ammonium, trifluoroacétate (composé 5+ composé 10 f)

P : 1,05 g. Rendement 83%. HPLC Kromasil C18 $CH_3CN/H_2O$ (0,5%TFA) 50/50 8,17 min. ESI $(M+H)^+$ = 531

Composé 12q: 1-(2-((cyclohexyl-propionylaxy-méthoxycarbonylméthyl)-carbamoyl)-3-phényl-propyldisulfanylméthyl)-3-méthylsulfanyl-propyl-ammonium, trifluororacétate (composé 5+ composé 10g)

P : 1,8 g. Rendement 78,2%. HPLC Kromasil C18 $CH_3CN/H_2O$ (0,5%TFA) 50/50 12,24 min. ESI $(M+H)^+$ = 571

Composé 12r : 3-méthylsulfanyl-1-(3-phényl-2-((phényl-propionyloxy-méthoxycarbonylméthyl)-carbamoyl)-propyldisulfanylméthyl)-propyl-ammonium, trifluoroacétate (composé 5+ composé 10h)

P : 0,98 g. Rendement 76,3%. HPLC Kromasil C18 $CH_3CN/H_2O$ (0,5%TFA) 50/50 11,25 min. ESI $(M+H)^+$ = 565

Exemple 13 : Synthèse des inhibiteurs mixtes pour lesquels $R_5$=hétérocycle

Le disulfure 5, 6 ou 7 (0,54 mmol) est dissous dans 5 mL de $CH_2Cl_2$ et l'aminothiadiazole de l'exemple 11 (1,2 eq), le TBTU (O-Benzotriazol-1-yl-N,N,N',N'-tetramethyluroniumtetrafluoroborate) (3eq) et la DIEA (diisopropylethylamine) (3eq) sont successivement ajoutés. Le mélange est agité pendant 30 minutes à température ordinaire (température ambiante, environ 20°C). Le solvant est évaporé sous vide et le résidu repris dans l'acétate d'éthyle. La phase organique est lavée à l'acide citrique, à l'eau, au NaCl saturé et séchée sur $Na_2SO_4$. Après filtration et évaporation sous vide, on obtient un solide blanc.
Le compose obtenu est dissous dans l'acide formique et le mélange est agité 1h à température ambiante. L'excès d'acide formique est évaporé sous vide. Le résidu est repris par l'éther et donne un précipité blanc.

Composé 13a= 3-(2-amino-4methylsulfanyl-butyldisulfanyl)-2-benzyl-N-(5-ethyl-(1,3,4)thiadiazol-2-yl)-propionamide

P=256 mg (Rdt 75%)

Exemple 14 : Changement de contre-ion.

Les composés de l'exemple 12 et de l'exemple 13 (1 mmol) sont solubilisés dans 9 ml de AcOEt distillé. La phase organique est lavée par 12 ml $NaHCO_3$ 0,1N. La phase organique est ensuite séchée et évaporée sous pression réduite. Le produit est repris dans AcOEt (3 ml), refroidie à 0°C et 1 eq de l'acide AH choisi dans 3 ml de AcOEt est ajouté. Le solvant est évaporé et le produit est précipité à froid dans un mélange $Et_2O$/Hexane.
(A= phosphate, chlorhydrate, acétate, méthanesulfonate, borate, lactate, fumarate, succinate, hémisuccinate, citrate, tartrate, hémitartrate, maléate, ascorbate, hemifumarate, hexanoate, heptanoate, hippùrate, hydrocinnamate, phénylglyoxylate, nicotinate).

Exemple 15 : Résultats pharmacologiques - composés selon l'invention

Le composé 15 de formule suivante a été testé dans les différentes tests biologiques ci-dessous.

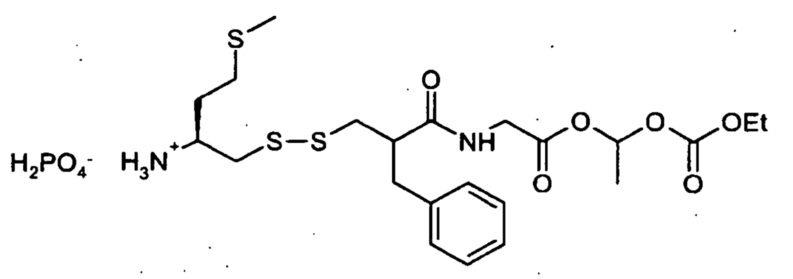

Test de la plaque chaude : il concerne le réflexe de lèchement et de saut de la souris sur une plaque chauffé à 52°C (mesure de la latence du saut dans les exemples donnés). Les résultats sont exprimés en % d'effet possible maximum (%MPE), c'est-à-dire en pourcentage d'analgésie, en utilisant l'équation suivante :

$$\% MPE = \frac{(\text{Temps de latence mesuré} - \text{temps de latence témoin})}{(\text{Temps de latence maximum} - \text{temps de latence témoin})}$$

Temps de latence maximum = 240 secondes.
Les résultats sont exprimés en terme de moyenne ± s.e.m. Les différences observées sont considérés comme étant significatives lorsque les valeurs de P sont inférieures à 0,05. Le test utilisé est le test ANOVA avec un test de comparaison multiple.

a- Réponses antinociceptives observées après injection par voie intraveineuse (iv) du composé 15 dans le test de la plaque chaude (52°C, réponse par saut) chez des souris mâles (n=10).

Le composé 15 est dissous dans le mélange eau/mannitol (50 mg/mL). Les temps de latence des sauts sont déterminés 10 min après les injections par voie intraveineuse.

Tableau 2

| Dose mg/kg | % analgésie |
|---|---|
| 10 | 33,4±7,5★★ |
| 20 | 59,2±9,6★★★ |
| 40 | 90,2±7,0 ★★★ |

Les résultats (tableau 2) montrent que le composé 15 présente une action analgésique, qui est dose-dépendante.
★★ P<0,01; ★★★P<0,001 *versus* véhicule.
La dose efficace 50, ED50, est de 16,1 mg/kg.
ED50 est la dose (en g/kg de corps) qui donne l'effet recherché chez 50% de la population chez qui elle est testée.

b- Effet antinociceptif du composé 15 (100 mg/kg per os) sur le seuil de vocalisation lors de la pression de la patte d'un rat (mâle Sprague Dawley) ayant une patte enflammée, l'inflammation étant induite par l'injection intraplantaire de carragénine

Le composé 15 et un véhicule (Ethanol/polyéthylène glycol (PEG) 400/eau, 10/40/50) sont administrés 180 min après une injection intraplantaire de carragénine (1% en solution saline).

Tableau 3

| | Seuil de vocalisation (g) |
|---|---|
| Seuil basal | 298,8±18,7 |
| Patte enflammée + véhicule | 205,5±17,8 |
| Patte enflammée + composé 15 | 292,5±20,1 ★★★ |

La ligne de base pour le seuil de vocalisation lors de la pression de la patte est mesurée avant inflammation (B) et pour la patte enflammée 20 min après injection du composé 15 ou du véhicule. Les résultats sont donnés dans le tableau 3 et exprimés en moyenne ± s.e.m., n = 10. Les différences observées sont considérées comme étant significatives lorsque les valeurs de P sont inférieures à 0,05. Ces résultats montrent que le composé 15 est efficace dans le traitement de la douleur inflammatoire neurogénique. ★★★P<0,001 *versus* véhicule.

c- Réponses antinociceptives observées après injection *iv* du composé 15 dissous dans éthanol/surfactant/eau (10/10/80) dans le test de la plaque chaude (52°C, réponse par saut) chez des souris mâles OF1 et comparaison des réponses en fonction de la nature du véhicule.

Le composé 15 est dissous dans un mélange Ethanol/Cremophor®EL/eau. Les temps de latences du saut

sont déterminés 10 min après les injections intraveineuses. Les résultats sont donnés dans la figure 1.
La réponse dose-dépendante fournit une réponse analgésique avec une dose efficace 50, DE 50 = 1,9 ± 0,4 mg/kg. Cette valeur est proche de celle observée avec la morphine (DE=1,3 ± 0,2 mg/kg).★ ★P<0,01 *versus* véhicule. ★ ★ ★ P<0,001.
Le composé 15 est également soluble dans des solvants du type éthanol /tween®/eau, qui sont fréquemment utilisés pour l'administration par voie intraveineuse chez l'homme
Dans ces solvants, les différences ne sont pas significativement différentes de celles observées avec le Cremophor®EL. Ceci est illustré dans le tableau 4 (temps de latence avant les sauts déterminés 10 min après les injections) à un même concentration de 2,5 mg/kg i.v.. ★ ★ ★*P*<0,001 *versus* véhicule.

Tableau 4

| | % analgésie |
|---|---|
| Cremophor EL | 56,2±6,9 ★ ★ ★ |
| Tween 80 | 44,7±6,2 ★ ★ ★ |
| Tween 20 | 48,4±10,2★ ★ ★ |

d- Effet antinociceptif du composé 15 injecté per os 20 min avant le test de la plaque chaude : a) courbe dose-réponse ; b)cinétique de l'effet après administration p.o de 200 m/kg.

Le composé 15 est dissous dans un mélange Ethanol/PEG400/eau 10/40/50. Les résultats, donnés sur les figures 2a et 2b, montrent que le composé 15 présente un effet analgésique dose-dépendant (2a), DE50 = 135 mg/kg, et que l'effet analgésique du composé 15 est très important juste après l'administration et perdure, avec un effet moins important, au moins deux heures (2b).
★*P<0,05;* ★ ★*P<0,01;* ★ ★ ★*P*<0,001 *versus* véhicule.
# *P*<0,05; # # # *P*<0,001 *versus* composé 15.

e- Réponses antinociceptives comparatives observées après injection iv du composé 15 *(n = 14-17)* ou du compose A (*n* = 8-14), 5 mg/kg, dans le test de la plaque chaude (52˚C, réponse par saut) chez des souris mâles. Le composé A est le composé de formule :

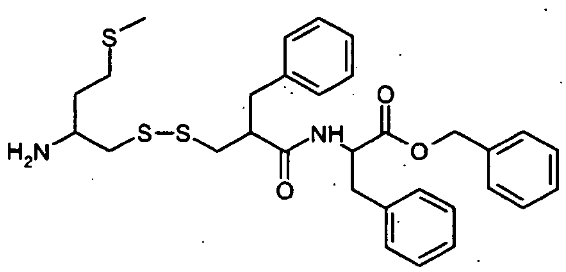

qui a été décrit dans la demande de brevet antérieure WO 91/02718 (exemple 7). C'est un inhibiteur mixte de la néprilysine et de l'aminopeptidase, qui présente des propriétés analgésiques. Il est peu soluble dans les solvants aqueux ou hydrophiles.
Les composés 15 et A sont dissous dans un mélange Ethanol/GremophorEL/eau, 10/10/80. Les temps de latence du saut sont mesurés 10 min après les injections intraveineuses.

Tableau 5

| | % analgésie |
|---|---|
| Composé 15 | 87,6±4,6★ ★, ### |
| Composé A | 51,1±7,6★ ★ |

Les résultats donnés dans le tableau 5 montrent qu'à des doses identiques (faibles afin de permettre l'administration du composé A par voie i.v.), le composé 15 est plus actif que le composé A.
★ ★*P<0,01* versus véhicule, # # # *P*<0,001 *versus* composé A.

f- Analgésie induite par la morphine ou le composé 15 injecté par voie i.v. chez la souris (test de la plaque chaude 52˚C)

L'analgésie produite par la morphine ou le composé 15 chez des souris (OF1 mâles) dans le test de la plaque chaude (52˚C) a été déterminée. La morphine dans $H_2O$ (NaCl, 9/1000) ou le composé 15, dissous dans le véhicule EtOH/cremophor EL/eau (10/10/80), est injecté par i.v.. Les résultats sont déterminés 10 minutes après l'injection, le temps de latence est de 240 secondes (équation donnée aux exemples 15 et 17).
Les résultats sont reproduits dans le tableau 6 suivant :

Tableau 6

| Dose (mg/kg) | 1 | 2,5 | 5 |
|---|---|---|---|
| % analgésie composé 15 | 46,4±5,6★★★ | 56,3±6,9★★★ | 87,6±4,6★★★ |
| % analgésie morphine | 45,5±4,5★★★ | 66±6,0 ★ ★ ★ | 100 ★ ★ ★ |
| ★ ★ ★ P<0,001, test Anova. | | | |

On constate que le composé 15 a des effets analgésiques comparables à ceux obtenus avec la morphine. Ceci est en accord avec les résultats d'une autre expérience effectuée dans des conditions identiques (Figure 1 exemple 15c).

Exemple 16 - Effet analgésique du $\Delta^9$ THC seul

L'analgésie produite par le $\Delta^9$ THC chez des souris (OF1 mâles) dans le test de la plaque chaude (52˚C) a été déterminée. Le $\Delta^9$ THC, dissous dans le véhicule EtOH/cremophor EL/eau (10/10/80), est injecté par i.v.. Les résultats sont déterminés 10 minutes après l'injection, le temps de latence est de 240 secondes (équation donnée aux exemples 15 et 17).
Les résultats sont reproduits dans le tableau 7 suivant :

Tableau 7

| Dose (mg/kg) | 0,188 | 0,375 | 0,75 | 1,5 |
|---|---|---|---|---|
| % analgésie | 0,4 ± 3,9 | 18,2 ± 6,4 | 41,2 ± 12,7 ★ ★ | 57,2 ± 10,9 ★ ★ ★ |
| ★ ★ P<0,01 ; ★ ★ ★ P<0,001 | | | | |

On constate que le $\Delta^9$ THC produit un effet analgésique dose-dépendant. Des valeurs d'analgésie significatives sont observées pour des doses de 0,75 mg/kg et 1,5 mg/kg. A des doses plus faibles, le pourcentage d'analgésie n'est pas significatif.

Exemple 17- Effets analgésiques des composés selon l'invention + $\Delta^9$ THC

Les expériences décrites pour démontrer l'importance de la synergie d'action des composés de l'invention associés au $\Delta^9$ THC ont été effectuées chez les rongeurs (rats et souris) grâce à des tests antinociceptifs classiquement utilisés dans l'industrie pharmaceutique pour mettre en évidence ce type de propriétés à savoir :

- le test de la plaque chaude (Eddie et Leimbach, J.Pharmacol. Exp. Ther. 107,385-389, 1953) chez la souris
- le test du retrait de la queue chez le rat (D'Amour et Smith, J. Pharmacol. Exp. Ther.72,74-79, 1941)
- le test de la nage forcée (test de Porsolt) chez la souris (Porsolt Arch.Int. Pharmacodyn.229,327,1977).

Pour la réalisation des tests centraux (plaque chaude, retrait de la queue), les teneurs :

- en cannabinoïdes ($\Delta^9$ THC) seront préférentiellement comprises entre 0,3 et 0,5 mg/kg,
- en les composés selon l'invention (composé 15) seront préférentiellement comprises entre 1 et 2 mg/kg.

Les proportions relatives sont dépendantes de la stimulation nociceptive.

Le $\Delta^9$ THC utilisé dans les exemples qui suivent est le produit commercial acheté chez Sigma-Aldrich (T2386).
L'inhibiteur mixte utilisé dans les exemples qui suivent est le composé 15 décrit précédemment (exemple 15).

Mise en solution des composés

Tous les composés ont été solubilisés dans le mélange éthanol/cremophor 80/ eau =10/ 10/80.
Pour les expériences de potentialisation (synergie), on utilise des doses de $\Delta^9$ THC et d'inhibiteurs qui, prises séparément, n'induisent pas de réponses significatives.

Mode d'administration.

Les différents composés sont administrés, en mélange dans la même seringue, par voie iv dans la queue du rat ou de la souris.

Animaux

Les souris utilisées dans ces tests sont les souris OF1 mâles.
Les rats utilisés dans ces tests sont des rats spague-Dawley mâles.

Tests pharmacologiques

- Test de la plaque chaude :

Ce test concerne le réflexe de lèchement et de saut de la souris sur une plaque chauffée à 52°C. Les résultats sont exprimés en pourcentage d'effet possible maximum (%MPE) c'est à dire en pourcentage d'analgésie, en utilisant l'équation suivante :

$$\%\,MPE = \frac{\text{(Temps de latence mesuré - temps de latence témoin)}}{\text{(Temps de latence maximum – temps de latence témoin)}}$$

Temps de latence maximum = 240 s

Les résultats sont exprimés en termes de moyenne +/- sem. Les différences observées sont considérées comme étant significatives lorsque les valeurs de P sont inférieures à 0,05.

- Test du retrait de la queue:

Ce test concerne le réflexe de retrait de la queue du rat, stimulée par la chaleur radiante émanant d'une source lumineuse focalisée sur une partie déterminée de la queue. Les résultats sont exprimés comme dans l'expérience précédente par la mesure d'un pourcentage d'analgésie selon la même équation. Le temps de latence maximum est fixé arbitrairement à 15 secondes.

- Test de la nage forcée:

Ce test mesure le temps d'immobilisation d'une souris placée dans un bain d'eau à 21-23°C, dont elle ne peut pas échapper. La durée d'immobilisation reflète une forme de dépression, la souris ne se débattant plus pour échapper au milieu hostile. Les résultats sont exprimés en durée d'immobilisation. Le temps maximum d'immobilisation est de 4 min.
Pour des besoins de démonstration de la synergie de l'association du composé 15 + $\Delta^9$THC, une courbe dose-réponse du composé 15 seul et du $\Delta^9$ THC seul sont effectuées dans le solvant éthanol/cremophorEL/$H_2O$ (1/1/8) car le $\Delta^9$ THC n'est utilisable seul par voie i.v à concentration élevée que dans ces conditions (exemple 16). L'histogramme (figure 3) des réponses analgésiques (plaque chaude) montre clairement la potentialisation très importante de l'effet analgésique de l'association $\Delta^9$-THC+composé 15 par rapport à l'un ou à l'autre des produits seuls.

a : Réponse antinociceptive induite par l'injection d'un mélange du composé 15 et du $\Delta^9$ THC par voie iv chez la souris OF1 mâle dans le test de la plaque chaude (figure 3).

Le mélange composé 15 (0,4 mg / kg) et $\Delta^9$ THC (0,375 mg/kg) est injecté dans le véhicule éthanol/Tween 80/eau (10/10/80). Les souris pèsent 25-30 grammes. Ce mélange de solvants est compatible avec une administration par voie iv chez l'homme et chez l'animal.
La latence du saut est déterminée 10 minutes après l'injection intraveineuse. Les résultats sont exprimés en pourcentage d'analgésie, en utilisant l'équation définie précédemment. Ils sont donnés sur la figure 3.
La différence observée est considérée significative pour une valeur de $p \leq 0,05$ - ANOVA 1 facteur suivit d'un test de comparaison multiple *** : $p \leq 0,01$ versus contrôle, ### :$p \leq 0,01$ versus groupe composé 15 et $\Delta^9$ THC.

b : Réponse antinociceptive induite par l'injection d'un mélange du composé 15 et du $\Delta^9$ THC par voie iv dans le test du retrait de la queue du rat Sprague-Dawley mâle. Le mélange composé 15 (5mg/kg) et $\Delta^9$ THC (0,375 mg/kg) est injecté dans le véhicule éthanol / Tween 80/eau (10/10/80). La latence du retrait de la queue est déterminée avant injection (pré-test) et 10 minutes après l'injection intraveineuse (test).

Le temps de latence est de 15 secondes. Les rats pèsent 260-300 grammes.
Les résultats sont donnés dans le tableau 8.

Tableau 8 : Test du retrait de la queue.

| | % analgésie |
|---|---|
| véhicule | -3,9±3,3 |
| Composé 15 | -4,2±1,9 |
| $\Delta^9$ THC | 23,3+2,4 ★★★ |
| Composé 15 ± $\Delta^9$ THC | 3 9,2±7,7 ★★, ★★★, # |
| ★★★: p < 0,001 versus véhicule, ★★: < 0,001 versus composé 15, # : p<0,05 versus $\Delta^9$ THC | |

La différence observée est considérée significative pour une valeur de $p \leq 0,05$. ANOVA deux facteurs suivi d'un test de comparaison multiple : ★★★: $p \leq 0,001$, ★★: $p \leq 0,01$ versus groupe véhicule ;# :$p \leq 0,05$ versus groupe $\Delta^9$ THC ; ★★★$p \leq 0,001$, ★★$p \leq 0,01$ versus groupe composé 15.

c : Réponse de type antidépresseur induite par injection d'un mélange du composé 15 et du $\Delta^9$ THC par voie iv dans le test de la nage forcée chez la souris OF1 mâle.

Le mélange composé 15 (5mg/kg) et $\Delta^9$ THC (0,375 mg/kg) est injecté dans le véhicule éthanol / Tween 80/eau (10/10/80) par voie intraveineuse 10 minutes avant le test.
La durée d'immobilisation totale est mesurée pendant 4 minutes.
Les résultats sont donnés sur le tableau 9. Les souris pèsent environ 25-30 grammes. La différence observée est considérée significative pour une valeur de $p \leq 0,05$. ANOVA 1 facteur suivi d'un test de comparaison multiple: ★ ★ ★ : $p \leq 0,001$, * :$p \leq 0,05$ versus groupe véhicule ;### :$p \leq 0,001$ versus groupe composé 15 / $\Delta^9$ THC ; et groupe $\Delta^9$ THC.

Tableau 9 : Test de dépression, nage forcée.

| | Mobilisation, (secondes) |
|---|---|
| véhicule | 230,7±4,5 |
| Composé 15 | 180±8,4 ★★★ |
| $\Delta^9$ THC | 206±6,1 |
| Composé 15 / $\Delta^9$ THC | 137,4±8,8 ★★★ , ### |
| ★★★: p < 0,001 versus controle, ### : p<0,001 1 versus THC et composé 15. | |

Conclusion :

On constate que l'administration d'une faible dose de cannabinoïde, le $\Delta^9$ THC, c'est à dire à des concentrations inférieures à 0,5 mg/kg par administration iv chez la souris, permet de potentialiser les réponses antinociceptives ou anti-dépressives induites par le composé 15.
Dans ces conditions, la synergie est très clairement mise en évidence lorsqu'on compare les effets analgésiques obtenus dans le test de la plaque chaude chez la souris en comparant les courbes doses-réponses pour le $\Delta^9$ THC et le composé 15 d'une part et l'effet produit par des doses sub-anaigésiques de ces deux composés (Figure 3).
On peut remarquer que les facteurs d'amplification dose active $\Delta^9$ THC seul/dose active $\Delta^9$ THC+composé 15, et inversement, dose active composé 15 seul/dose active composé 15+$\Delta^9$ THC, sont dans les deux cas très supérieurs à 10 et difficilement calculables précisément puisque les doses utilisées du composé 15 et $\Delta^9$ THC sont inactives lorsque ces deux molécules sont utilisées seules à ces mêmes doses.
L'intensité de la réponse antinociceptive donnée par l'association du composé selon l'invention (inhibiteur mixte NEP/APN ; par exemple le composé 15) et du $\Delta^9$ THC, les deux substances étant administrées à très faibles doses où elles sont dépourvues d'activité, indique l'existence d'une synergie d'action entre enképhalines endogènes (protégées par les composé selon l'invention) et le $\Delta^9$ THC. Ceci est corroboré par une analyse isobolographique des réponses pharmacologiques.

## Revendications

**1.** Composés répondant à la formule (I) suivante :

$$H_2N\text{-}CH(R_1)\text{-}CH_2\text{-}S\text{-}S\text{-}CH_2\text{-}CH(R_2)\text{-}CONH\text{-}R_5$$

dans laquelle :

$R_1$ représente :

- une chaîne hydrocarbonée, saturée ou insaturée, linéaire ou ramifiée, comportant de 1 à 6 atomes de carbones, éventuellement substituée par :

  * un radical OR, SR ou S(O)R, dans chacun de ces radicaux R représente un hydrogène, une chaîne hydrocarbonée linéaire ou ramifiée de 1 à 4 atomes de carbone, un radical phényle ou benzyle,
  * un radical phényle ou benzyle,

- un radical phényle ou benzyle éventuellement substitué par :

  * 1 à 5 halogènes, notamment le fluor,
  * un radical OR, SR ou S(O)R, dans chacun de ces radicaux R ayant la même signification que précédemment,

- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde ;

$R_2$ représente :

- un radical phényle ou benzyle, éventuellement substitué par:

  * 1 à 5 atomes d'halogènes notamment le fluor,
  * un radical OR ou SR, dans chacun de ces radicaux R ayant la même signification que précédemment,
  * un groupement amino éventuellement mono- ou di-substitué par un groupement aliphatique de 1 à 6 atomes de carbones,
  * un cycle aromatique à 5 ou 6 atomes,

- un hétérocycle aromatique à 5 ou 6 atomes, l'hétéroatome étant un oxygène, un azote ou un soufre,
- un groupe méthylène substitué par un hétérocycle à 5 ou 6 atomes, aromatique ou saturé, l'hétéroatome

étant un oxygène, un azote ou un soufre, les atomes d'azote et de soufre étant éventuellement oxydés sous forme de N-oxyde ou de S-oxyde.

$R_5$ représente un radical $CH(R_3)$-$COOR_4$ dans lequel
$R_3$ représente :

- un hydrogène,
- un groupement OH ou OR, R ayant la même signification que précédemment
- une chaîne hydrocarbonée saturée (alkyle), linéaire ou ramifiée, comportant de 1 à 6 atomes de carbone, éventuellement substitué par un radical OR ou SR, dans chacun de ces radicaux R a la même signification que précédemment,
- un radical phényle ou benzyle, éventuellement substitué par :

  * 1 à 5 halogènes notamment le fluor,
  * un radical OR ou SR, R ayant la même définition que précédemment.

et
$OR_4$ représente

- un radical glycolate $OCH_2COOR'$ ou lactate $OCH(CH_3)COOR'$, dans chacun de ces radicaux R' représente

  • une chaîne hydrocarbonée saturée (alkyle) de 1 à 6 atomes de carbone, linéaire ou ramifiée éventuellement substituée par un groupement alkoxy en C1 à C3, de préférence un groupe alkyle en C1-C4 éventuellement substitué par un groupe méthoxy
  • un groupement cycloalkyle de C5 à C8, de préférence un groupe cycloalkyle en C5-C6,
  • un groupement phényle, benzyle, hétéroaryle, alkylhétéroaryle

- un groupement OCH(R")O(CO)OR' ou OCH(R")O(CO)R', dans chacun de ces radicaux R' a la même signification que précédemment et R" représente

  • un atome d'hydrogène
  • une chaîne alkyle en C1-C6 linéaire ou ramifiée éventuellement substituée par un groupement alkoxy de C1 à C3, de préférence un groupe alkyle en C1-C4 éventuellement substitué par un groupe méthoxy
  • un groupe cycloalkyle de C5 à C8, de préférence un groupe cycloalkyle en C5-C6,
  • un groupement phényle, benzyle, hétéroaryle, alkylhétéroaryle,

- un radical triglycéride $OCH(CH_2OCOR')_2$ ou $OCH_2$-$CH(OCOR')$-$CH_2OCOR'$ dans chacun de ces radicaux R' ayant la même signification que précédemment,
- un radical glycoside tel que D-glucose, $\beta$-D-glucopyranose, $\alpha$ -ou $\beta$-galactopyranose,
- un radical sulfonate $OCH_2CH_2(SO_2)CH_3$,
- un radical $OCH(CH_2OH)_2$ ;

ainsi que les sels d'addition desdits composés (I) avec des acides minéraux ou: organiques pharmaceutiquement acceptables.

**2.** Composés selon la revendication 1, **caractérisés en ce que** $R_1$ représente un radical alkyle ayant de 1 à 4 atomes de carbone substitué par un radical SR, R ayant la même signification que précédemment, en particulier R représente une chaîne hydrocarbonée saturée linéaire ou ramifiée de 1 à 4 atomes de carbone.

**3.** Composés selon l'une quelconque des revendications précédentes, **caractérisés en ce que** le radical $R_2$ représente un radical benzyle ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde.

**4.** Composés selon l'une quelconque des revendications précédentes, **caractérisés en ce que** le radical $R_3$ représente un atome d'hydrogène ou un radical alkyle ayant de 1 à 6 atomes de carbone substitué par un radical OH ou SH.

**5.** Composés selon l'une quelconque des revendications précédentes, **caractérisés en ce que** le radical $OR_4$ repré-

sente un groupement OCH(R")O(CO)OR’ ou OCH(R")O(CO)R’,

- le radical R’ représentant une chaîne alkyle en C1-C4, en particulier le radical éthyle, et
- le radical R" représentant un radical méthyle, $CH(CH_3)_2$, cyclohexyle ou phényle.

**6.** Composés selon l’une quelconque des revendications précédentes, **caractérisés en ce qu’**ils sont choisis parmi les composés suivants:

1-(2-(1-(2,3-diacétoxypropoxycarbonyl)-éthylcarbamoyl)-3-thiophèn-3-ylpropyl disulfanylméthyl)-3-méthylsulfanylpropyl-amine.
1-(2-(1-(2-méthanesulfonyléthoxycarbonyl)-éthylcarbamoyl)-3-thiophèn-3-ylpropyl disulfanylméthyl)-3-méthylsulfanylpropyl- amine.
1-(2-(1-(1-éthoxycarbonyloxyéthoxycarbonyl))-éthylcarbamoyl)-3-thiophèn-3-yl-propyl disulfanylméthyl)-3-méthylsulfanylpropyl- amine.
1-(2-(1-éthoxycarbonylméthyloxycarbonyléthyl carbamoyl) -3-thiophèn-3-yl-propyl disulfanylméthyl)-3-méthylsulfanylpropyl- amine.
1-(2-(1-(1-éthoxycarbonyloxyéthoxycarbonyl)-2-hydroxypropylcarbamoyl)-3-thiophèn-3-ylpropyldisulfanylméthyl)-3-méthylsulfanylpropyl- amine.
1-(2-(1-(2-acétoxy-1-acétoxyméthyléthoxycarbonyl)-éthylcarbamoyl)-3-thiophén-3-yl propyldisulfanylméthyl)-3-méthylsulfanylpropyl- amine.
1-(2-(1-(2-hydroxy-1-hydroxyméthyléthoxycarbonyl)-éthylcarbonyl)-3-thiophèn-3-yl propyldisulfanylméthyl)-3-méthylsulfanylpropyl- amine.
1-(2-(1-(3,4,5,6-tétrahydroxytétrahydropyran-2-ylméthoxycarbonyl)-éthylcarbamoyl)-3-thiophèn-3-yl-propyldisulfanylméthyl)-3-méthylsulfanylpropyl-amine.
1-(2-(1-(1-éthoxycarbonyloxy-éthoxycarbonyl)-2-hydroxypropylcarbamoyl)-3-phényl propyldisulfanylméthyl)-3-méthylsulfanylpropyl-amine.
1-(2-(1-(2- acétoxy- 1- acétoxyméthyl- éthoxycarbonyl)- 2- hydroxypropylcarbamoyl)- 3- phénylpropyldisulfanylméthyl)-3-méthylsulfanylpropyl-amine.
1-(2-((1-éthoxycarbonyloxy-éthoxycarbonylméthyl)-carbamoyl)-3-phényl-propyl disulfanylméthyl)-3-méthylsulfanylpropyl-amine.
1(2-((1-éthoxycarbonyloxy-2-méethyl-propoxycarbonylméthyl)-carbamoyl)-3-phényl-propyldisulfanylméthyl)-3-méthylsulfanyl-propyl-amine
1-2-((cyctohexyl-ethoxycarbonyloxy-méthoxycarbonylméthyl)-carbamoyl)-3-phényl-propyldisulfanylméthyl)-3-méthylsulfanyl-propyl-amine
1-(2-((éthoxycarbonyloxy- phényl- méthoxycarbonylméthyl)-carbamoyl)- 3- phényl- propyldisulfanylméthyl)- 3-méthylsuafanyl-propyl-amine
3- méthylsulfanyl- 1-(3- phényl- 2-((1- propionyloxy- éthoxycarbonylméthyl)-carbamoyl)-propyldisulfanylméthyl)-propyl-amine
1-(2-((2-méthyl-1-propionyloxy-propoxycarbonylméthyl)-carbamoyl)-3-phényl-propyldisulfanylméthyl)-3-méthylsulfanyl-propyl-amine
1-(2-((cyclohexyl-propionyloxy-méthoxycarbonylméthyl)-carbamoyl)-3-phényl-propyldisulfanylméthyl)-3-méthylsulfanyl-propyl-amine
3-méthylsulfanyl-1-(3-phényl-2-((phenyl-propionyloxy-méthoxycarbonylméthyl)-carbamoyl)-propyldisulfanylméthyl)-propyl-amine

**7.** A titre de médicaments, les composés de formule (I) selon l’une quelconque des revendications précédentes.

**8.** Composition pharmaceutique, **caractérisée en ce qu’**elle comprend au moins un composé de formule (I), selon l’une quelconque des revendications 1 à 6, et un excipient pharmaceutiquement approprié, en particulier un excipient approprié pour une administration par voie orale, nasale ou intraveineuse.

**9.** Composition pharmaceutique selon la revendication 8, **caractérisée en ce qu’**elle est destinée au traitement de la dépression et des différents types de douleur, tels que la douleur aigue, la douleur nociceptive, la douleur inflammatoire et la douleur neurogénique.

**10.** Utilisation d’un composé de formule (I) selon l’une quelconque des revendications 1 à 6, pour la préparation d’un médicament destiné au traitement de la dépression et de la douleur.

**11.** Composition pharmaceutique selon la revendication 8 ou 9, **caractérisée en ce qu'**elle comprend en outre au moins un dérivé de cannabinoïdes, en particulier le $\Delta^9$-tetrahydrocannabinol.

**12.** Utilisation d'une combinaison d'au moins un composé de formule (I) selon l'une quelconque des revendications 1 à 6 et d'au moins un dérivé de cannabinoïdes, en particulier le $\Delta^9$-tetrahydrocannabinol, pour la préparation d'un médicament destiné au traitement de la dépression et de la douleur.

**13.** Composition pharmaceutique comprenant

i) au moins un composé de formule (I), selon l'une quelconque des revendications 1 à 6
ii) au moins un dérivé de cannabinoïdes

en tant que produits de combinaison pour une utilisation simultanée, séparée ou étalée dans le temps.

**14.** Composition pharmaceutique selon la revendication 8, 9 ou 11, **caractérisée en ce qu'**elle comprend en outre de la morphine ou l'un de ses dérivés.

**15.** Utilisation d'une combinaison d'au moins un composé de formule (I) selon l'une quelconque des revendications 1 à 6 et de morphine ou d'un de ses dérivés, pour la préparation d'un médicament destiné au traitement de la dépression et de la douleur.

**Claims**

**1.** Compounds of following formula (I):

$$H_2N\text{-}CH\ (R_1)\ \text{-}CH_2\text{-}S\text{-}S\text{-}CH_2\text{-}CH\ (R_2)\ \text{-}CONH\text{-}R_5$$

wherein:

$R_1$ represents:

- a hydrocarbon chain, saturated or unsaturated, linear or branched, comprising from 1 to 6 carbon atoms, optionally substituted by:

  * an OR, SR or S(O)R radical, wherein in each of these radicals R represents a hydrogen, a linear or branched hydrocarbon chain of 1 to 4 carbon atoms, a phenyl or benzyl radical,
  * a phenyl or benzyl radical,

- a phenyl or benzyl radical optionally substituted by:

  * 1 to 5 halogens, notably fluorine,
  * an OR, SR or S(O)R radical, wherein in each of these radicals R is defined as above,

- a methylene radical substituted by a 5- or 6-atom heterocycle, aromatic or saturated, having as a heteroatom an atom of nitrogen or sulfur, optionally oxidized in the form of N-oxide or S-oxide;

$R_2$ represents:

- a phenyl or benzyl radical, optionally substituted by:

  * 1 to 5 halogen atoms, notably fluorine,
  * an OR or SR radical, wherein in each of these radicals R is defined as above,
  * an amino group optionally mono- or disubstituted by an aliphatic group of 1 to 6 carbon atoms,
  * a 5- or 6-atom aromatic ring,

- a 5- or 6-atom aromatic heterocycle, the heteroatom being oxygen, nitrogen or sulfur,
- a methylene group substituted by a 5- or 6-atom heterocycle, aromatic or saturated, the heteroatom being

oxygen, nitrogen or sulfur, the nitrogen and sulfur atoms possibly being oxidized in the form of N-oxide or S-oxide,

$R_5$ represents a $CH(R_3)$-$COOR_4$ radical wherein:

$R_3$ represents:

- hydrogen,
- an OH or OR group, with R as defined above,
- a saturated hydrocarbon chain (alkyl), linear or branched, comprising from 1 to 6 carbon atoms, optionally substituted by an OR or SR radical, wherein in each of these radicals R is defined as above,
- a phenyl or benzyl radical, optionally substituted by:

    * 1 to 5 halogens, notably fluorine,
    * an OR or SR radical, with R as defined above, and,

$OR_4$ represents:

- an $OCH_2COOR'$ glycolate or $OCH(CH_3)COOR'$ lactate radical, wherein in each of these radicals R' represents:

    * a saturated hydrocarbon chain (alkyl) with 1 to 6 carbon atoms, linear or branched and optionally substituted by a C1-C3 alkoxy group, preferably a C1-C4 alkyl group optionally substituted by a methoxy group,
    * a C5-C8 cycloalkyl group, preferably a C5-C6 cycloalkyl group,
    * a phenyl, benzyl, heteroaryl, alkylheteroaryl group,

- an OCH(R")O(CO)OR' or OCH(R")O(CO)R' group, wherein in each of these radicals R' is defined as above and R" represents:

    * a hydrogen atom,
    * a C1-C6 alkyl chain, linear or branched, optionally substituted by a C1-C3 alkoxy group, preferably a C1-C4 alkyl group optionally substituted by a methoxy group,
    * a C5-C8 cycloalkyl group, preferably a C5-C6 cycloalkyl group,
    * a phenyl, benzyl, heteroaryl, alkylheteroaryl group,

- an $OCH\ (CH_2OCOR')_2$ or $OCH_2$-CH (OCOR') -$CH_2OCOR'$ triglyceride radical, wherein in each of these radicals R' is defined as above,
- a glycoside radical such as D-glucose, β-D-glucopyranose, α- or β-galactopyranose,
- an $OCH_2CH_2(SO_2)CH_3$ sulfonate radical,
- an $OCH(CH_2OH)_2$ radical;

as well as additive salts of the aforesaid compounds (I) with pharmaceutically acceptable mineral or organic acids.

2. Compounds according to claim 1, **characterized in that** $R_1$ represents an alkyl radical having from 1 to 4 carbon atoms substituted by a SR radical, with R defined as above, notably with R representing a saturated hydrocarbon chain, linear or branched, with 1 to 4 atoms of carbon.

3. Compounds according to any one of the preceding claims, **characterized in that** radical $R_2$ represents a benzyl radical or a methylene radical substituted by a 5- or 6-atom heterocycle, aromatic or saturated, having as a heteroatom an atom of nitrogen or sulfur, optionally oxidized in the form of N-oxide or S-oxide.

4. Compounds according to any one of the preceding claims, **characterized in that** radical $R_3$ represents a hydrogen atom or an alkyl radical having from 1 to 6 carbon atoms substituted by an OH or SH radical.

5. Compounds according to any of the preceding claims, **characterized in that** radical $OR_4$ represents an OCH(R") O(CO)OR' or OCH(R")O(CO)R' group, wherein:

- the R' radical represents a C1-C4 alkyl chain, notably an ethyl radical, and
- the R" radical represents a methyl, $CH(CH_3)_2$, cyclohexyl or phenyl radical.

**6.** Compounds according to any one of the preceding claims, **characterized in that** said compounds are selected among the following compounds:

• 1-(2-(1-(2,3-diacetoxypropoxycarbonyl)-ethylcarbamoyl)-3-thiophen-3-ylpropyldisulfanylmethyl)-3-methylsulfanylpropyl-amino,
• 1-(2-(1-(2-methanesulfonylethoxycarbonyl)-ethylcarbamoyl)-3-thiophen-3-ylpropyldisulfanylmethyl)-3-methylsulfanylpropyl-amino,
• 1-(2-(1-(1-ethoxycarbonyloxyethoxycarbonyl))-ethylcarbamoyl)-3-thiophen-3-yl-propyldisulfanylmethyl)-3-methylsulfanylpropyl-amino,
• 1-(2-(1-ethoxycarbonylmethyloxycarbonylethylcarbamoyl)-3-thiophen-3-yl-propyldisulfanylmethyl)-3-methylsulfanylpropyl-amino,
• 1-(2-(1-(1-ethoxycarbonyloxyethoxycarbonyl)-2-hydroxypropylcarbamoyl)-3-thiophen-3-ylpropyldisulfanylmethyl)-3-methylsulfanylpropyl-amino,
• 1-(2-(1-(2-acetoxy-1-acetoxymethylethoxycarbonyl)-ethylcarbamoyl)-3-thiophen-3-ylpropyldisulfanylmethyl)-3-methylsulfanylpropyl amino,
• 1-(2-(1-(2-hydroxy-1-hydroxymethylethoxycarbonyl)-ethylcarbamoyl)-3-thiophen-3-ylpropyldisulfanylmethyl)-3-methylsulfanylpropyl-amino,
• 1-(2-(1-(3,4,5,6-tetrahydroxytetrahydropyran-2-ylmethoxycarbonyl)-ethylcarbamoyl)-3-thiophen-3-yl-propyldisulfanylmethyl)-3-methylsulfanylpropyl-amino,
• 1-(2-(1-(1-ethoxycarbonyloxy-ethoxycarbonyl)-2-hydroxypropylcarbamoyl)-3-phenylpropyldisulfanylmethyl)-3-methylsulfanylpropyl-amino,
• 1-(2-(1-(2-acetoxy-1-acetoxymethyl-ethoxycarbonyl)-2-hydroxypropylcarbamoyl)-3-phenylpropyldisulfanylmethyl)-3-methylsulfanylpropyl-amino,
• 1-(2-((1-ethoxycarbonyloxy-ethoxycarbonylmethyl)-carbamoyl)-3-phenyl-propyldisulfanylmethyl)-3-methylsulfanylpropyl-amino,
• 1-(2-((1-ethoxycarbonyloxy-2-methyl-propoxycarbonylmethyl)-carbamoyl)-3-phenyl-propyldisulfanylmethyl)-3-methylsulfanyl-propyl-amino,
• 1-(2-((cyclohexyl-ethoxycarbonyloxy-methoxycarbonylmethyl)-carbamoyl)-3-phenyl-propyldisulfanylmethyl)-3-methylsulfanyl-propyl-amino,
• 1-(2-((ethoxycarbonyloxy-phenyl-methoxycarbonylmethyl)-carbamoyl)-3-phenyl-propyldisulfanylmethyl)-3-methylsulfanyl-propyl-amino,
• 3-methylsulfanyl-1-(3-phenyl-2-((1-propionyloxy-ethoxycarbonylmethyl)-carbamoyl)-propyldisulfanylmethyl)-propyl-amino,
• 1- (2-((2-methyl-1-propionyloxy-propoxycarbonylmethyl)-carbamoyl)-3-phenyl-propyldisulfanylmethyl)-3-methylsulfanyl-propyl-amino,
• 1-(2-((cyclohexyl-propionyloxy-methoxycarbonylmethyl)-carbamoyl)-3-phenyl-propyldisulfanylmethyl)-3-methylsulfanyl-propyl-amino,
• 3-methylsulfanyl-1-(3-phenyl-2-((phenyl-propionyloxy-methoxycarbonylmethyl)-carbamoyl)-propyldisulfanylmethyl)-propyl-amino.

**7.** As drugs, compounds of formula (I) according to any one of the preceding claims.

**8.** A pharmaceutical composition, **characterized in that** it comprises at least one compound of formula (I), according to any one of the claims 1 to 6, and a pharmaceutically suitable excipient, notably an excipient suitable for administration by oral, nasal or intravenous route.

**9.** The pharmaceutical composition according to claim 8, **characterized in that** it is to treat depression and the various types of pain, such as acute pain, nociceptive pain, inflammatory pain and neurogenic pain.

**10.** The use of a compound of formula (I) according to any one of the claims 1 to 6, for preparing a drug to treat depression and pain.

**11.** The pharmaceutical composition according to claim 8 or 9, **characterized in that** it comprises in addition at least one cannabinoid derivative, notably $\Delta^9$-tetrahydrocannabinol.

**12.** The use of a combination of at least one compound of formula (I) according to any one of the claims 1 to 6 and at least one cannabinoid derivative, notably $\Delta^9$-tetrahydrocannabinol, for preparing a drug to treat depression and pain.

**13.** A pharmaceutical composition comprising:

i) at least one compound of formula (I), according to any one of the claims 1 to 6,
ii) at least one cannabinoid derivative,

as combination products for simultaneous, separate or staggered use.

**14.** The pharmaceutical composition according to claim 8, 9 or 11, **characterized in that** it comprises in addition morphine or a derivative of same.

**15.** The use of a combination of at least one compound of formula (I) according to any one of the claims 1 to 6 and morphine or a derivative of same, for preparing a drug to treat depression and pain.

## Patentansprüche

**1.** Verbindungen gemäß der folgenden Formel (I):

$$H_2N\text{-}CH(R_1)\text{-}CH_2\text{-}S\text{-}S\text{-}CH_2\text{-}CH(R_2)\text{-}CONH\text{-}R_5$$

wobei:

$R_1$ folgendes repräsentiert:

- eine gesättigte oder ungesättigte, lineare oder verzweigte Kohlenwasserstoffkette umfassend 1 bis 6 Kohlenstoffatome, gegebenenfalls substituiert durch:

  * ein OR-, SR- oder S(O)R-Radikal, wobei in jedem dieser Radikale R einen Wasserstoff, eine lineare oder verzweigte Kohlenwasserstoffkette mit 1 bis 4 Kohlenstoffatomen, ein Phenylradikal oder ein Benzylradikal repräsentiert,
  * ein Phenylradikal oder ein Benzylradikal,

- ein Phenylradikal oder ein Benzylradikal, gegebenenfalls substituiert durch:

  * 1 bis 5 Halogene, insbesondere Fluor,
  * ein OR-, SR- oder S(O)R-Radikal, wobei in jedem dieser Radikale R dieselbe Bedeutung hat wie oben,

- ein Methylenradikal substituiert durch einen aromatischen oder gesättigten Heterocyclus mit 5 oder 6 Atomen, der als Heteroatom ein Stickstoffatom oder ein Schwefelatom aufweist, gegebenenfalls oxidiert in Form eines N-Oxids oder eines S-Oxids;

$R_2$ folgendes repräsentiert:

- ein Phenylradikal oder ein Benzylradikal, gebenenfalls substituiert durch:

  * 1 bis 5 Halogenatome, insbesondere Fluor,
  * ein OR- oder SR-Radikal, wobei in jedem dieser Radikale R dieselbe Bedeutung hat wie oben,
  * eine Aminogruppe, gegebenenfalls mono- oder di-substituiert durch eine aliphatische Gruppe mit 1 bis 6 Kohlenstoffatomen,
  * einen aromatischen Ring mit 5 oder 6 Atomen,

- einen aromatischen Heterocyclus mit 5 oder 6 Atomen, wobei das Heteroatom ein Sauerstoff, ein Stickstoff oder ein Schwefel ist,
- eine Methylengruppe, substituiert durch einen aromatischen oder gesättigten Heterocyclus mit 5 oder 6 Atomen, wobei das Heteroatom ein Sauerstoff, ein Stickstoff oder ein Schwefel ist, und wobei das Stickstoffoder Schwefelatom gegebenenfalls oxidiert ist in Form eines N-Oxids oder eines S-Oxids,

$R_5$ ein Radikal $CH(R_3)$-$COOR_4$ repräsentiert, wobei
$R_3$ folgendes repräsentiert:

- einen Wasserstoff,
- eine OH- oder OR-Gruppe, wobei R dieselbe Bedeutung hat wie oben,
- eine lineare oder verzweigte gesättigte Kohlenwasserstoffkette (Alkyl) umfassend 1 bis 6 Kohlenstoffatome, gegebenenfalls substituiert durch ein OR- oder SR-Radikal, wobei in jedem dieser Radikale R dieselbe Bedeutung hat wie oben,
- ein Phenylradikal oder ein Benzylradikal, gegebenenfalls substituiert durch:

    * 1 bis 5 Halogene, insbesondere Fluor,
    * ein OR- oder SR-Radikal, wobei R dieselbe Bedeutung hat wie oben,

und
$OR_4$ folgendes repräsentiert:

- ein Glycolat-Radikal $OCH_2COOR'$ oder ein Lactat-Radikal $OCH(CH_3)COOR'$, wobei in jedem dieser Radikale R' folgendes repräsentiert:

    • eine lineare oder verzweigte gesättigte Kohlenwasserstoffkette (Alkyl) mit 1 bis 6 Kohlenstoffatomen, gegebenenfalls substituiert durch eine C1-C3-Alkoxygruppe, vorzugsweise eine C1-C4-Alkylgruppe, gegebenenfalls substituiert durch eine Methoxygruppe,
    • eine C5-C8-Cycloalkylgruppe, vorzugsweise eine C5-C6-Cycloalkylgruppe,
    • eine Phenyl-, Benzyl-, Heteroaryl- oder Alkylheteroarylgruppe,

- eine OCH(R")O(CO)OR'-Gruppe oder eine OCH(R")O(CO)R'-Gruppe, wobei in jedem dieser Radikale R' dieselbe Bedeutung hat wie oben und R" folgendes repräsentiert:

    • ein Wasserstoffatom,
    • eine lineare oder verzweigte C1-C6-Alkylkette, gegebenenfalls substituiert durch eine C1-C3-Alkoxygruppe, vorzugsweise eine C1-C4-Alkylgruppe, gegebenenfalls substituiert durch eine Methoxygruppe,
    • eine C5-C8-Cycloalkylgruppe, vorzugsweise eine C5-C6-Cycloalkylgruppe,
    • eine Phenyl-, Benzyl-, Heteroaryl- oder Alkylheteroarylgruppe,

- ein Triglyceridradikal $OCH(CH_2OCOR')_2$ oder $OCH_2$-$CH(OCOR')$-$CH_2OCOR'$, wobei in jedem dieser Radikale R' dieselbe Bedeutung hat wie oben,
- ein Glycosidradikal wie z.B. D-Glucose, β-D-Glucopyranose oder α-oder β-Galactopyranose,
- ein Sulfonatradikal $OCH_2CH_2(SO_2)CH_3$,
- ein Radikal $OCH(CH_2OH)_2$;

sowie die Additionssalze der Verbindung (I) mit pharmazeutisch akzeptablen Mineralsäuren oder organischen Säuren.

2. Verbindungen nach Anspruch 1, **dadurch gekennzeichnet, dass** $R_1$ ein Alkylradikal mit 1 bis 4 Kohlenstoffatomen repräsentiert, welches durch ein SR-Radikal substituiert ist, wobei R dieselbe Bedeutung hat wie oben, und wobei R insbesondere eine lineare oder verzweigte gesättigte Kohlenwasserstoffkette mit 1 bis 4 Kohlenstoffatomen repräsentiert.

3. Verbindungen nach einem der vorangehenden Ansprüche, **dadurch gekennzeichnet, dass** das Radikal $R_2$ ein Benzylradikal repräsentiert oder ein Methylenradikal, welches substituiert ist durch einen aromatischen oder gesättigten Heterocyclus mit 5 oder 6 Atomen, der als Heteroatom ein Stickstoffatom oder ein Schwefelatom aufweist, gegebenenfalls oxidiert in Form eines N-Oxids oder S-Oxids.

4. Verbindungen nach einem der vorangehenden Ansprüche, **dadurch gekennzeichnet, dass** das Radikal $R_3$ ein Wasserstoffatom oder ein Alkylradikal mit 1 bis 6 Kohlenstoffatomen repräsentiert, welches durch ein OH- oder SH-Radikal substituiert ist.

**5.** Verbindungen nach einem der vorangehenden Ansprüche, **dadurch gekennzeichnet, dass** das Radikal $OR_4$ eine OCH(R")O(CO)OR'-Gruppe oder eine OCH(R")O(CO)R'-Gruppe repräsentiert,

- das Radikal R' eine C1-C4-Alkylkette repräsentiert, insbesondere das Ethylradikal, und
- das Radikal R" ein Methylradikal, ein $CH(CH_3)_2$-Radikal, ein Cyclohexylradikal oder ein Phenylradikal repräsentiert.

**6.** Verbindungen nach einem der vorangehenenden Ansprüche, **dadurch gekennzeichnet, dass** sie aus den folgenden Verbindungen ausgewählt sind:

1-(2-(1-(2,3-diacetoxypropoxycarbonyl)-ethylcarbamoyl)-3-thiophen-3-ylpropyldisulfanylmethyl)-3-methylsulfanylpropyl-amin,
1-(2-(1-(2-methanesulfonylethoxycarbonyl)-ethylcarbamoyl)-3-thiophen-3-ylpropyldisulfanylmethyl)-3-methylsulfanylpropyl-amin,
1-(2-(1-(1-ethoxycarbonyloxyethoxycarbonyl))-ethylcarbamoyl)-3-thiophen-3-yl-propyldisulfanylmethyl)-3-methylsulfanylpropyl-amin,
1-(2-(1-ethoxycarbonylmethyloxycarbonylethyl carbamoyl)-3-thiophen-3-yl-propyldisulfanylmethyl)-3-methylsulfanylpropyl-amin, 1-(2-(1-(1-ethoxycarbonyloxyethoxycarbonyl)-2-hydroxypropylcarba-moyl)-3-thiophen-3-ylpropyldisulfanylmethyl)-3-methylsulfanylpropyl-amin,
1-(2-(1-(2-acetoxy-1-acetoxymethylethoxycarbonyl)-ethylcarbamoyl)-3-thiophen-3-ylpropyldisulfanylmethyl)-3-methylsulfanylpropyl-amin,
1-(2-(1-(2-hydroxy-1-hydroxymethylethoxycarbonyl)-ethylcarbamoyl)-3-thiophen-3-ylpropyldisulfanylmethyl)-3-methylsulfanylpropyl-amin,
1-(2-(1-(3,4,5,6-tetrahydroxytetrahydropyran-2-ylmethoxycarbonyl)-ethylcarbamoyl)-3-thiophen-3-yl-propyldisulfanylmethyl)-3-methylsulfanylpropyl-amin,
1-(2-(1-(1-ethoxycarbonyloxy-ethoxycarbonyl)-2-hydroxypropylcarba-moyl)-3-phenylpropyldisulfanylmethyl)-3-methylsulfanylpropyl-amin,
1-(2-(1-(2-acetoxy-1-acetoxymethyl-ethoxycarbonyl)-2-hydroxypropylcarbamoyl)-3-phenylpropyldisulfanylmethyl)-3-methylsulfanylpropylamin,
1-(2-((1-ethoxycarbonyloxy-ethoxycarbonylmethyl)-carbamoyl)-3-phenyl-propyldisulfanylmethyl)-3-methylsulfanylpropyl-amin,
1(2-((1-ethoxycarbonyloxy-2-meethyl-propoxycarbonylmethyl)-carbamoyl)-3-phenyl-propyldisulfanylmethyl)-3-methylsulfanyl-propyl-amin,
1-2-((cyclohexyl-ethoxycarbonyloxy-methoxycarbonylmethyl)-carbamoyl)-3-phenyl-propyldisulfanylmethyl)-3-methylsulfanyl-propyl-amin,
1-(2-((ethoxycarbonyloxy-phenyl-methoxycarbonylmethyl)-carbamoyl)-3-phenyl-propyldisulfanylmethyl)-3-methylsulfanyl-propyl-amin,
3-methylsulfanyl-1-(3-phenyl-2-((1-propionyloxy-ethoxycarbonylmethyl)-carbamoyl)-propyldisulfanylmethyl)-propyl-amin,
1-(2-((2-methyl-1-propionyloxy-propoxycarbonylmethyl)-carbamoyl)-3-phenyl-propyldisulfanylmethyl)-3-methylsulfanyl-propyl-amin,
1-(2-((cyclohexyl-propionyloxy-methoxycarbonylmethyl)-carbamoyl)-3-phenyl-propyldisulfanylmethyl)-3-methylsulfanyl-propyl-amin,
3-methylsulfanyl-1-(3-phenyl-2-((phenyl-propionyloxy-methoxycarbonylmethyl)-carbamoyl)-propyldisulfanylmethyl)-propyl-amin.

**7.** Die Verbindungen der Formel (I) nach einem der vorangehenden Ansprüche als Medikamente.

**8.** Pharmazeutische Zusammensetzung, **dadurch gekennzeichnet, dass** sie mindestens eine Verbindung der Formel (I) nach einem der Ansprüche 1 bis 6 umfasst sowie einen pharmazeutisch geeigneten Hilfsstoff, insbesondere einen Hilfsstoff, der für eine orale, nasale oder intravenöse Verabreichung geeignet ist.

**9.** Pharmazeutische Zusammensetzung nach Anspruch 8, **dadurch gekennzeichnet, dass** sie zur Behandlung von Depression und von verschiedenen Arten von Schmerz bestimmt ist, wie z.B. akuter Schmerz, nozizeptiver Schmerz, Entzündungsschmerz und neurogener Schmerz.

**10.** Verwendung einer Verbindung der Formel (I) nach einem der Ansprüche 1 bis 6 zur Herstellung eines Medikaments, das zur Behandlung von Depression und von Schmerz bestimmt ist.

**11.** Pharmazeutische Zusammensetzung nach Anspruch 8 oder 9, **dadurch gekennzeichnet, dass** sie ferner mindestens ein Derivat von Cannabinoiden umfasst, insbesondere $\Delta^9$-Tetrahydrocannabinol.

**12.** Verwendung einer Kombination von mindestens einer Verbindung der Formel (I) nach einem der Ansprüche 1 bis 6 und mindestens einem Derivat von Cannabinoiden, insbesondere $\Delta^9$-Tetrahydrocannabinol, zur Herstellung eines Medikaments, das zur Behandlung von Depression und von Schmerz bestimmt ist.

**13.** Pharmazeutische Zusammensetzung, umfassend

i) mindestens eine Verbindung der Formel (I) nach einem der Ansprüche 1 bis 6,
ii) mindestens ein Derivat von Cannabinoiden,

als Kombinationsprodukt für eine simultane, getrennte oder zeitlich verteilte Anwendung.

**14.** Pharmazeutische Zusammensetzung nach Anspruch 8, 9 oder 11, **dadurch gekennzeichnet, dass** sie ferner Morphin oder eines seiner Derivate umfasst.

**15.** Verwendung einer Kombination von mindestens einer Verbindung der Formel (I) nach einem der Ansprüche 1 bis 6 und von Morphin oder einem seiner Derivate zur Herstellung eines Medikaments, das zur Behandlung von Depression und von Schmerz bestimmt ist.

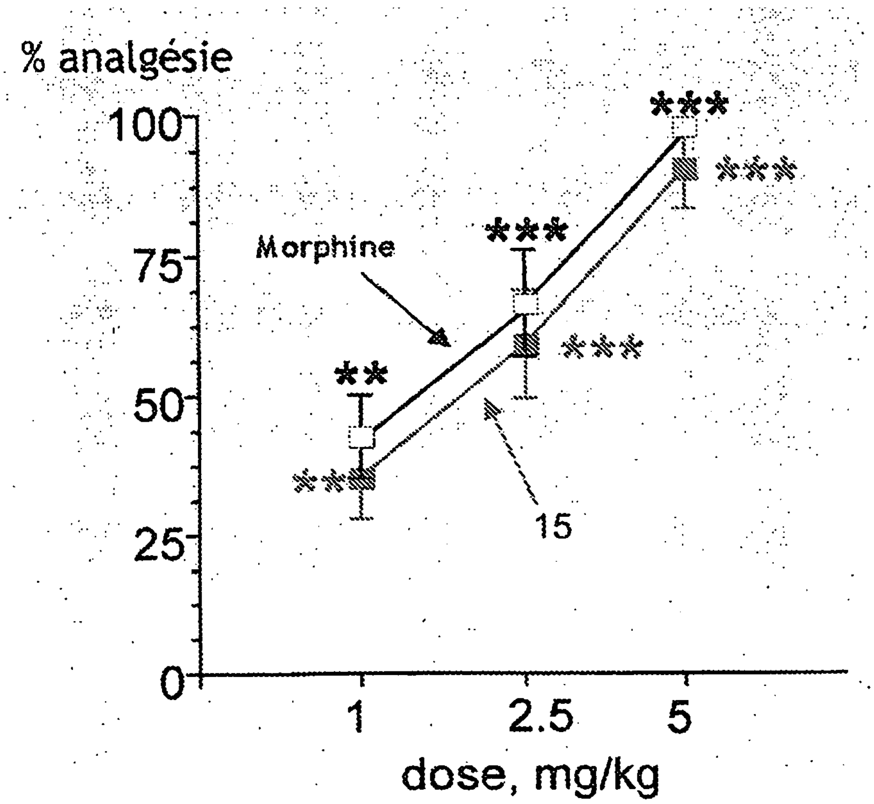


FIG. 1

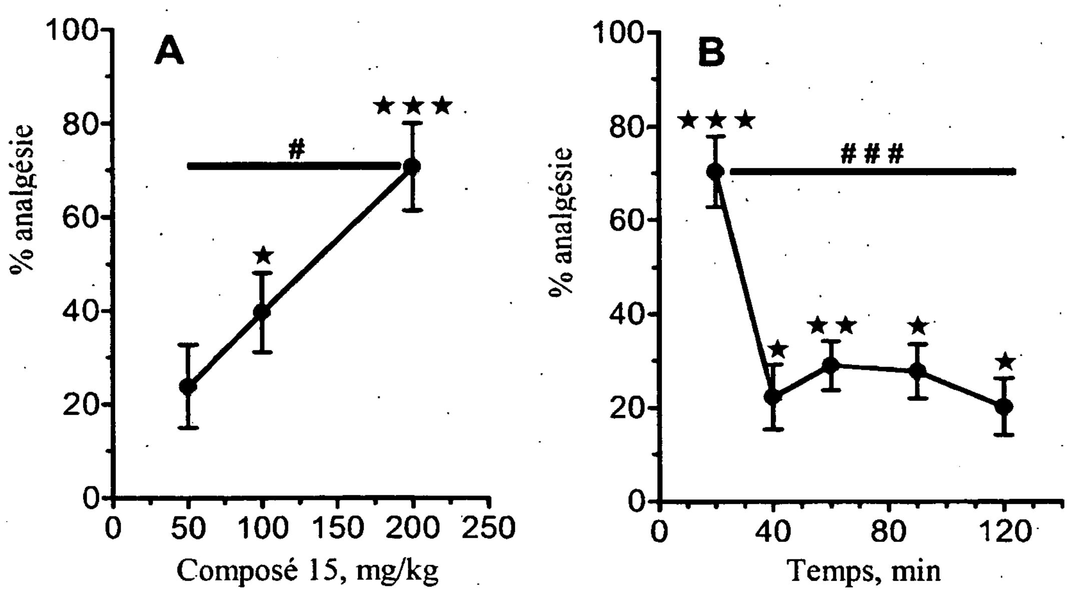
A
100
80
60
40
20
0
% analgésie
#
★★★
★
0
50
100
150
200
250
Composé 15, mg/kg
B
100
80
60
40
20
0
% analgésie
★★★
###
★
★★
★
★
0
40
80
120
Temps, min

FIG. 2A et 2B

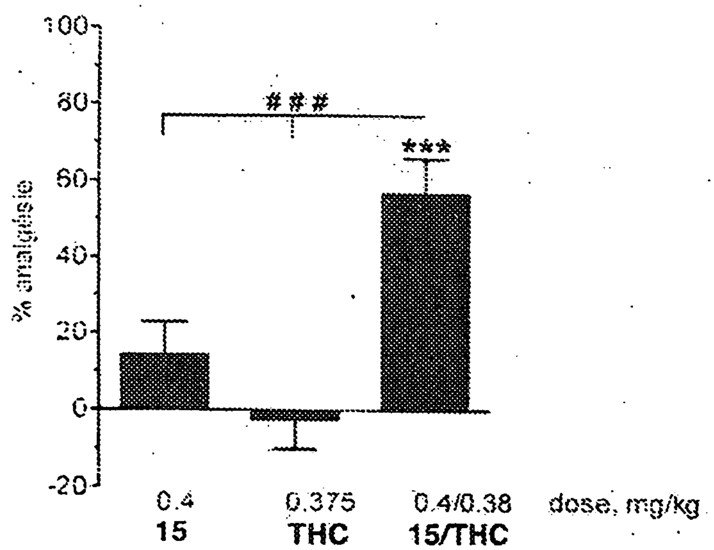
100
80
60
40
20
0
-20
% analgesia
###
***
0.4
15
0.375
THC
0.4/0.38
15/THC
dose, mg/kg

FIG. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2518088
- FR 2605004
- FR 2755135
- FR 2777780
- FR 2651229
- WO 9102718 A


### Littérature non-brevet citée dans la description


- *Nature,* 1978, vol. 276, 523
- *Eur.J.Pharmacol.,* 1985, vol. 117, 233
- *Pharmacological review.,* 1993, vol. 45, 87-146
- *Eur. J. Phamacol.,* 1984, vol. 102, 525-528
- *Eur.J.Pharmacol.,* 1989, vol. 165, 199-207
- *Eur.J.Pharmacol.,* 1991, vol. 192, 253-262
- *Brain Research,* 1989, vol. 497, 94-101
- *J.Med.Chem.,* 2000, vol. 43, 1398-1408
- *J.Med Chem.,* 2001, vol. 44, 3523-3530
- *J.Pharm.Exp.Ther.,* 1992, vol. 261, 181-190
- *Pain,* 1997, vol. 73, 383-391
- *J.Med.Chem.,* 1992, vol. 35, 1259
- *Ber.,* 1924, vol. 57, 1116
- *Biochemistry,* 1977, vol. 16, 5484
- *Bioorg. Med.Chem.Let.,* 1993, vol. 3, 2681
- **Mitsunobu.** *Synthesis,* 1981, 1-28
- *Current Opinion in Investigational Drugs,* 2004, vol. 5, 748
- **Piomelli et al.** *TIS,* 2000, vol. 21, 218-224
- **E.A. Carlini.** the good and the bad effects of (-)transdelta-9-etrahydrocannabinol Δ9THC on humans. *Toxicon,* 2004, vol. 44, 461-467
- **Campbell F.A. et al.** Are cannabinoids an effective and safe treatment option in the management of pain ? A quantitative systemic review. *Br. Med. J.,* 2001, vol. 323, 12-16
- **Piomelli et al.** *TIPS,* 2000
- *J.Med.Chem.,* 1992, vol. 35, 2473
- *Bioor.Med.Chem.Let.,* 1993, vol. 3, 2681
- **Bentley ; McCrae.** *J.org.Chem,* 1971, vol. 35, 2082
- **Eddie ; Leimbach.** *J.Pharmacol. Exp. Ther.,* 1953, vol. 107, 385-389
- **D'Amour ; Smith.** *J. Pharmacol. Exp. Ther.,* 1941, vol. 72, 74-79